# EUROPEAN PATENT APPLICATION

(11) **EP 3 715 474 A2**
(43) Date of publication of application: **30.09.2020**
(21) Application number: 18881098.0
(22) Date of filing: 19.11.2018
(51) Int. Cl.: C12Q 1/6886

(54) **METHOD FOR ASSESSING PROGNOSIS OR RISK STRATIFICATION OF LIVER CANCER BY USING CPG METHYLATION VARIATION IN GENE**

(30) Priority: 22.11.2017 KR 20170156527; 09.11.2018 KR 20180137644
(71) Applicant: Lepidyne Co., Ltd., Seongdong-gu Seoul 04778 (KR)
(72) Inventor: KIM, Young Joon, Seoul 06024 (KR); KIM, Da Won, Seoul 06366 (KR); CHOI, Won Young, Seoul 04351 (KR); LEE, Jung Woo, Seoul 03722 (KR); JUNG, MinHyeok, Seongnam-si Gyeonggi-do 13600 (KR); HA, Jeong Sil, Seoul 06024 (KR); KIM, Ji Won, Seongnam-si Gyeonggi-do 13332 (KR); LEE, Yeon Su, Gimpo-si Gyeonggi-do 10085 (KR); HWANG, Jung Ah, Incheon 21378 (KR); KIM, Tae You, Seoul 06001 (KR); LIM, Yoo Joo, Seoul 06355 (KR)
(74) Representative: Vossius & Partner Patentanwälte Rechtsanwälte mbB
(86) International application number: PCT/KR2018/014207
(87) International publication number: WO 2019/103421

(57) **Abstract**

The present invention relates to a method for assessing the prognosis or risk stratification of liver cancer by using a clinical specimen mixed with a normal tissue, wherein at least one CpG site that shows a low methylation level in normal and blood tissues but a high methylation level in only a cancer tissue is measured for methylation level.

<110>	Yonsei University

<120>	A METHOD FOR ASSESSING RISK OF HEPATOCELLULAR CARCINOMA USING CPG
	METHYLATION STATUS OF GENOME

<130>	DP-2017-1051

<160>	44

<170>	KoPatentIn 3.0

<210>	1
<211>	552
<212>	DNA
<213>	Homo sapiens

<400>	1
tcgccctctg gctcggcacg gaggggggcg ctcagccttt ctggggcaaa tttagtaata	60

tgggacccga gccctcgacc cgaaatacgc ccgaggcatt tatcctaaaa aacgacaagg	120

tccgggcgcc cagcagaacg gcccggctcg accgcgcgca gcttgcaggc aggggggtgt	180

gcaggtcacc gcgccacccc ggcgagcaga gccgcggagg gcgccacgtc ggtgcgctgg	240

ccccgcccga gcggggcggg accttcctgt acccccggaa gcccccgcgg gcagctgggg	300

aggaaaccgc ggccacgcgc tcggggggcc cggctcggga agggcagtgc gcgcgcatgc	360

gttggggcgg ggcgcctggg acctgcgggc cccaggccca gcgcgccgcc agccggagtg	420

cccggcgccc gtcgaaaggc ccctgcgccg gttcaggacc cgcacccagc tacgctgcgg	480

agccccagct cgcagcaccc tcccacccac cgctcctggc tgcttttctc ctgagtctgc	540

ggggcggggt cg	552


<210>	2
<211>	1074
<212>	DNA
<213>	Homo sapiens

<400>	2
gcggcagcag gtgccggcag cgcggggacc gatcgatgga gagaaggcgg gcaagacgcc	60

gggaagcgca ttcctcctca accgagtgcc acaaccgccc tcccgaagtg ccccggggct	120

tcgagcatca cctcgcggta atccgggagg gtggagggat gcggctggac ccgggcgttg	180

cgtgctccac acagcgccca gcccgtgcca gccccgcgcc cacctctcca cgacgctcgt	240

gccgggatca gcgcgaagcc ccttccagtc cccgaagccc tcgcccgcgc ccgttctccc	300

ccagctcgcc ccctccagcc cgctgcgcct tgccgcagca tctccgggca ctctgaggct	360

gccgccggga cagggtcgga gcgccgcaga acccaccgaa acttcccagg ggggcaattc	420

aaaattcgcc ggacgcgtcg ccgccgcgcg cccctcggct cattcccttc cgcgcgcccg	480

cagccccagg ctctccctct ctcaggaccc cccagcgccc tgcgcggcga gaataggccc	540

ccaggtgcct cccggccccg ggggctgccg tcgcacgtcc gctcccgcag gggtcctcac	600

tccgccaatc gccgcggccg cgcgccctcg cgcacactca ccagcccgag ccggggcggc 660

catcttagcg ctcaccccgg ccccccgccc cccggttcgg cggccgcgac gacccggtgc 720

ggcggctacg acagccgtga cgcgcagcag gccccgcccc ctcccacagc cccacccctg 780

cgccggctct tcgcgggcac cgagaacctg ccggtggccg ccttccgcgc ctcgtggggg 840

ggtcggggcc acggacggtc cccggcgccg caagtgggtc tgcgcgaaca acaagcactg 900

cctccccggg cgggcttcgc acctgtagtg ccgtcgggac acgggagggt aaacccagcg 960

tgtcctgtgt gcctgtgagc cgcagaatca tccacggacg tcgttagtcc ttcctggaat 1020

ttctgcgatt tacacaacgt cgaattgttt ggcagaaacg cgtggcaaac tccg 1074

<210> 3
<211> 5293
<212> DNA
<213> Homo sapiens

<400> 3
acgcgccgag tttaagccct ttctatttcc ctttaacgct tccgcaaatg ccaagagaaa 60

tcgtaccacc gcagtgatat cattatttac atttaatttt taaaaattaa aactcaacag 120

ccacgcccat taagatgcag cgatgggcag ccccggccac agaggctgcg ggaggctgga 180

ggggttttgt cagccgcagt cacagccccg cggagctggc ggcatttcag ggcaggagac 240

gggtcccccg agcccccggc tgggcgctgc gggccttgcc cagggggcct ccggctccct 300

gaccccgcgt gacccacggg aggccccgcc gctccgcggg cggaattatt tcggatttct 360

ctttgcggtc ctagttcgga agaaactgct ttccaccgcg ggaagatctg gcgggatggt 420

gaccgaaggg cctccgtgca gcggatcaga cccggttcca ccggctgagc ccagggcggg 480

cctatgggat ccgctgatgc gcagagggac tttggaaata atcagagcga agccctcggc 540

caagcgggaa cgggtgcccg gtggcaacga gtacgtggcc ccaaagcggg aaaacggaag 600

aagaaaaacc tcccgcgggg actcgaggcg ggtacgcggc tcacccgccc tttcgggaac 660

ccccaagcgc gtccgaatcc gccccgaggc gaggcgggcc gggccgtacc tgctgctccg 720

tccccggctc cgtcccgggc tcctggcggc tgtcgctgcg gttccttccc gcgggccggg 780

ccccttccct gcgccttcgc cgcctcctcg cgcctgcccg gggcccgcag cctccgcacc 840

gggaacccgg aggacccgag gcgggcgcag gggcgaagcc ggggccgggg aggggccgcc 900

tcgctccggg ttcgagacgg aagaaacacg cggcgcaggc tccggagcga cggctccgac 960

ggggacccgt taaataattt attgatgata caaagcgact cgcgcccacc cggggccgcc 1020

cccggattct gcaaaaatag attcgccccc accccgcggg tcctcacaag gcgtcccccg 1080

cgccgccgcc gcacgggctg accagcgcca agttcgaggg tttgtgcttc ttgagcagcc 1140

gcgtgatctt ctcgtcgtcc gagttggggt ccaggggccg gttgtattcg tcgtcgtcct 1200

ccgcgtccga gccgcccacc ttcagcttct cggcgtccga gtcctgcttc ttcttggccg 1260

acgccatctc caccgcgtgc cgcttgcgcc acttggtccg gcggttctgg aaccagacct 1320

gggagtggac ggggcggtca ggcggccgcg gggcccgggg ctggcgctgg ggccgttcgc 1380

aggacgcggg cccccggctc tgctctcccg agccccgccg cgctcacctt cacctggctc 1440

tcggtcatgc ccagcgagta ggcgagacgc gcgcgctccg ggcccgccag gtacttggtc 1500

tgctcgaagg ttttctccag cgcgaagatc tgctggcccg agaaggtcgg gcgcgagtgc 1560

ttcttcttcc cgtccttgtc caggacgccg ccggccgggg ctgcaaggga ggggaaggga 1620

gggaggtcag cggccggcgg ggtccccctc cgcgcccacc cgccccgcac cccccgcgcg 1680

ggccactcac ccgggccagc cagacgcggg tccctccagg gcgcgccctg caccacgccg 1740

ggccagaaga tgggcggcgg ccccggcagc tcggccaggg gcttggggta gccgcgcgcc 1800

acagcggccg cgggcccgaa gtaaacgccg gcggacgacg cgagcccgtt gagccggggc 1860

agccccccca ggaggccccc gcccgccgcg cccacgggcc ggcccaggat gtcgctgatg 1920

ccgtgcgggg tcccgagcgg gagctgcgcg cccaggcccc ccagcgcggg cgccttgaag 1980

ccggccggac cctgcagcgc gtaggggaac agcgacgtct tcatctcggc catgttgtgc 2040

agcgcggcca gcggggcact gctcagcacg aacgcgcccg ggcggttagt gtccatgggc 2100

gccgccgccg ccggcccggg ctcccatccg ggccccgccg ccgccgcccc tgcccgccgg 2160

cccgggaagt ttgcgcgcgg cccgggcggg cgtcggctgc agcgcggggc gcggggcgcg 2220

gggggcgggc gggcggctcc ggcgcggggc gggcgggcgg gcggcggcgg cggcggctcc 2280

ggggccggtc ggagcggcgc cgcgcgggac ggacgcgctg ataacggggg ctccccgggg 2340

cgcggcgcgc gcgctgattg gctgcggacc ccgcggtccg gccattggcc ggcgcccccc 2400

ccccgcccgc gcgcccccgc cggccgcgca ctccatgaag ggcccattag cgcggcaggt 2460

gcctcccggg ctgtaaattc gccccgattt atctccccgg ggacgaaata aatccagctt 2520

ggatgggagt gtagttaggc aaaggttttc atgcgaaatc aggaaaaaat acgagaacgt 2580

attttattaa cggaaagaat gcagatttga ggacccgccc gcgcgctcgg agcgccccac 2640

tcggggaaga gtcccggccc gcgtcccagt cgcgactgct ccgccgcccg tggctggggt 2700

gggtgtgagc ggcagcggac gccggcgggg aacgcgctcc gggcaggtcg gggccacagg 2760

agggcggagg acgcgggcct cgagtccacg gaatccacgg gcctcacggc cgggcgaggc 2820

tcgcacggag ctgcctctgg tttcgccgac acgcggccgg cgcggtggag gagtgaggca 2880

ggcgggagcg ggcggggagg gcgggtcccg ggaccacacg cgcggctcgg cacttccccg 2940

tcatcggcct ccaggtctcc cgctgggggt cccccaggat gtgacctggg cccacgactt 3000

cgcccacggg ccgcctctcg cgaatccccg gccgggagaa cagagaccag gacggcctca 3060

gcgcggaagc cctgtccagg gcccgaacgt gggtgcgggc tgggggcgca gcggcagaaa 3120

cgcggcctta gacgcgcgcg gggggccggt gtcccccccg cccccacggc accgggagcc 3180

gctcgctcat ccatcccgca gaccgggcgg tgagatgact ccgagccccg cgcacggcgg 3240

ccgcgagcaa acgctccgac gtctgtggtg acgtctcgta ttgatttagg gacacggggc 3300

ggctgtggct gtggcccacg gctcgtgggg agcccgagtc tgtgcgcagg gaacgccgct 3360

gcgtggcctc tctcgggcct gtgccgcgga ggaaggcggc gcccggggtt cggggccggg 3420

gtctcacgtc cgccccctcg cccccctcca gcgtccgttt tcgttttgtg caggtcgagg 3480

cggggacttg gcgccgtcgg ccgctcctgg atggcggctt ggaaaagcca cctgcggcca 3540

aactccgggg cagtggtgcg gccgccgggt gtgtgcgcgc tcggggctgc cccggcggct 3600

tccggctcca ctgaggtgca gccccgcgtt cacggggggt tcgcttcccc cgtcgactcc 3660

gcactcgatt cgactgggat tgggattcga ttgggcgcgg ccgccccacc ggtgatcggc 3720

ccccgcggag cctggcccgg gaacccccag cgcccgtccg gccccgagac ccgccccggc 3780

ctgtcctgcg ccccgctccc tcggaggaga cacgaggaag ggccctcccg ggtcgggttc 3840

gggcccctcc cagcacccca aggcgacggc gcccgcgccc aaggctcggg ctctgagctg 3900

agacgcggga actgcggggc cggggggggc gggcaggggg agttgggggc gggggctgct 3960

gtcgccctgg gatcccccac tctgcgcggc cgggcagacc ctgggcgggc cgggaggggt 4020

gcgggtcgcc cgaactgagg cccaggaggc gcgcgcgggc ggaacggcgg gaggaagccg 4080

cgctcgggac aggctctggc tcttctcaca gcctggaggg gcggggcagg gggcgtcgct 4140

agcgctgttt tatggctgag gaaacatgga cctggaggcg ctgcgctgcg gcccgataaa 4200

ccctgggtct gtctgcgccc cccggtcctc cctgggctgc cgagggaacg cgtggggtcg 4260

cgggtggggc tggcgtgtca ctggctgcgg agcgcgcggc tctccagggc tcaggggcgc 4320

gggatgaggg gccagggtga gttgggggcg cagaggagcc gggtaagggg gggtccctct 4380

cccacgttct tgtaagccgt ggaagtcttg gcgcgctccg caggtgccgc gtcctacccg 4440

gctccccggc ctgcgcccca cgcgctttcc cgccctcctc ctcgcggagc tgcgcgtggg 4500

tccggtggct tcacctccta cgcttcccgg cgcccactcc gggccccgtc ccctctccgg 4560

cccccgcccg caccttttct cctgcgtccc ccggtgccgc cctctcggaa accaccgagg 4620

caactccccc tcctcccggg aaggtccgag cgcctccgac cgcgatgtct ttgcctgggc 4680

tccgccgccc ggcggccccg ctgcctggag aggtccgcga tgccacctcc tggcccgcga 4740

gcagatgtcc cgcgaggaag gctgccggca tcggcgccga cgctccgcgc tggaaaaccg 4800

agagcgcggg gtttggcaga ggccattcaa gtttgtttta ctcgttcagc ctgtatttgt 4860

ggggctccta ccgcgcccgg gctgtccggg gggtgcaccg tgaacgcagc gggctccggc 4920

ccgggcgcgg gcgggtcaga gcagcaaacg cgctcccccg gcacccccgc ggcgtctgca 4980

ggggagcggg cgacggggag gacggcgggg gtgtcggccc ggaaggagag ccggcctagg 5040

ccccggtgtc cccgcgctgg gggctgaggg gcgacggggg aactccacag agggaaggag 5100

cctgcgttcg ccttcggccg cccaggccat agagttcaca aactctgatt tatcactgag 5160

gtgacttgtc ccgcgcagcc ctaggcgagt tgccaagctg cccgccctga ccgcgccccc 5220

aggccggggg tctcctagca gttcccggca aaggccctgc attgtctttt ttcccgaagt 5280

gagcgcattc ccg 5293

<210> 4
<211> 2001
<212> DNA
<213> Homo sapiens

<400> 4
gcctcaccct ggaacaggga gtccagcagg tcctggttga cacagccggg gctggcgtgg 60

tggacaagga agcctggacc acagcagatg catgagtgca ggccccacag ccccccaggg 120

ggaggctggc ccagctccca aagccccggt gccaggggca gtgtgacccc gggcggcctc 180

acctataagc acggcggctg cccggcgcag ggggtcctgt ggactccgca ggtagccctg 240

ggtctggctc aggaagttgg gcacgtggcc tgggtatcgc tgaacctggg gacaaaaggg 300

ctagtggcag gacaggaggg ctgatcctga gtgcggagga ggctgcagag ctgaatccag 360

gggccggggt tccaggggag cccccagggc aggtggcatg gtcggagacc ttggacttgc 420

cccaccagta gcctatctgg tttggctgca gtagaaacgg ttgggggccc cggtgaaccc 480

tggaacaagt gggctgctga tcataccccc ttgcggtcac cttgcttccc ctactgacca 540

ggcggcagca gaggtggctc agggcctcgg ggctgtcata gtgggccacg gtgaccaact 600

cctccagcag gccccagcaa aaggcgtggt cacagcgggc cagggtccac tctgagctct 660

gggatagggg aagtgagccg ggtcaggggt ccaggaagta gaaaggcaaa aggtggggtg 720

ggaagagggg gagcaagggc atcgggtgag gggcagaaga gcccaggcca ggagactgga 780

ttgattctgc tcaagggaag agcagtagta acctggccgc ccgtcacacc tgccactgag 840

gtccttggga tgggtgagtc cctgacctgt aattgtcgga ggggaggcac ggtgggagtg 900

gtgagtgttg gatggcatag gggtgggatg gtgtcggggg ctgctgacct cagcagcgtc 960

cctgctgggg tcatgcaggc gcagcagcag cggcacgaga ctctgcagca ccagcttccg 1020

cagggggccg cggagcccca gccggagccc gccccggccc cggcgcacca gagtcccaag 1080

gagcccgacg gccgaggcgc ggattgagtc ccgtgtctgc gtgggagggc gcagtcaggg 1140

caggcggaga cagagagggg ctgcaagggt gggagggggc ggccagcgcg gagcgaggaa 1200

gcggcgggtc tagggaaggc tgctgactcg gtgtgatctg gggacaggga acagggcctg 1260

gagctggacc tggttgggaa gcctggagag cccctgcagg gggtggggct tgaagggatg 1320

gggtccggaa ggaaaagtcg agcggggagg agcttggcgg gacacggccc tggaggggcg 1380

gagctgggcg acagcaggcg ggaggggcgg gggcggtcag gagggaagaa atctgggacg 1440

gagacactgg ggggacgggg cctgggaggg agaaactgga ggggcggggc ggagcctggg 1500

aggtcagggc ctgggaggga cagactgaca gactgggggg cggggcatgg gaaggagaaa 1560

ctagaggggc gggcggggcc tgggaggcgg ggcctgggag ggagagactg taggggcggg 1620

gcggggcatg agagggagaa acaggagggg cggggcgggg cctgggaggg agactggggg 1680

cggggcctgg gagggagaga ctggaagggc ggggcggggc ctgagagggc ggggcctggg 1740

cgggagagac gggggcgggg cctgggaggg agagactgga agggcggggg cggtgacagc 1800

ggcaggggcg ggacccggag gcggggcgtt tgctcacgtc gtccagtagc ggagggaggc 1860

gcggtcccag ctccgcgctc aggagccgca caggcgcccg gggccgcagc aggagcctcc 1920

tcagggcgcc cagcgctgca cccacgagcc gcgcgtcgcc ttcgcccagt gcgcccagga 1980

gcgccggcag cagcgtgctc a 2001

<210> 5
<211> 619
<212> DNA
<213> Homo sapiens

<400> 5
ccgtccggga ctcgggggga ggcgcgctgg gtggtccggc agccgggggc gggcggtagc 60

ctgcaggcgt aattggcatg cacgccgttg tagctgagac cgcttaataa agcattacat 120

atctcaccgc ttccatattt cattacctca cgcggagcct gtgagagggc cctaatggga 180

gtcagctgtg tttttacttt ctgttgtcgg ccgggacggg tttctctgcg gattctttga 240

aatgaaataa tgtgatgcac gccgcgataa gggccggcct gtaatgaggc ccaggccgcc 300

gggcggctgc tattgctcca ggtgtcgcgt atttgggctg cgaggacaag gaggaggagg 360

gggcggcgcc ggaggatcgg gggggagggg gaagtcgcga ggggcagggg gtgggagaag 420

gcggagggag gaggcagggg gcagggggcg ggcggaagag gggaggaagg agggggcggc 480

gggccgcggc aggccaggcg ggagaagccg gagacagaga gaggacgggg acagtggcgg 540

cctgcagagc cctaggaacc cgggttcaaa tcctgcccgc cagcgtgaga gagcgtttgc 600

ccgcccaggc ggtccaccg 619

<210> 6
<211> 2001
<212> DNA
<213> Homo sapiens

<400> 6
ggctgccatt tctcatgggg ttcgagccat aagatgccag gtgtcagtca cctcagaaga 60

gagggttttg tttgtatttg gtggggttct tccgggggta ccgaggctga ggactgatgg 120

atcgaggcgg gtggatcact tgagttcgga gttcgagacc agcctggcca acatggcgaa 180

accctgtctc tactaaaaat acaaaaatta gccaggtgaa aaattagccg ggcatggtgg 240

tgcttgtctg taatcccagc tacatatgta attccagcta ctcaggaagc tgaggcagga 300

gaattgcttg aacctgcggg gcggaggttg cagtgagccg agatcgtgcc actacactcc 360

cgtgagggag cagcctaggg actaaggccc gctggctccg cgagatcagc caggcccgca 420

tcatccccca attacctgca gagggcgccg cagacacaga gagggcgagg ccgaggccga 480

ggccatctaa gcttctggga agggggtccc aaagggaggc cccgagctgg agtccagggg 540

gcttggaagg aagaggtgag gacagcagaa aaggaaacgc aaattaaaag aagggaacga 600

aggctgggcg cggtggctta cgcctgtaag tccagcactt tgggaggccg agtcgggagg 660

atcgcttgag gtcaggagtt caagaccagc ctggccaaca tggtgaaacg ccgtctctac 720

taaaaataca aaaaaattag ccaggagtgg tggcaggtgc ctgtcatccc agctactccg 780

caggctgagg tgggaggatc gcttgagccc gggaggtggg ggttgcagtg agccgagatc 840

gcgccactgc actccaggct gggtgataga gtgagaccca gtctcaaaaa aagagaagga 900

aaagagagaa gggatggtgg cggtggggga ggaaggcggt tcttgtaatg atccgagagg 960

agaccgcagc accggttacg ccctcgaacc tcggcctcct catctgcaga atggacgcag 1020

tccacaccgc gtgctgtgga agggttggag cgccggttta catgacacag ggctatcggc 1080

taagggggcg ctgggaacgt ggaaggtgct ctatggtggg gcgctgtagg gtgctcttgg 1140

gcagtagggt tggagtcaaa tctgggttga agtccaacct aggttgaagt cctggctgcc 1200

acctgacctc tgcccctcag tttgctcatc agtaaaatgg ggttaaggag gctgcctcgc 1260

ccagctccac ggagccggag gtgatgaagg tcctggaaga gcagaattca gaacccgagc 1320

tttgggcggc ggagcaggac agggcgcggg tgggcgcggc ctccgggagg ccagcacgag 1380

ggggagcggc ggggcccgga cacacccagc cagaaggagg aggccgaccc cgcgccgact 1440

ccgcagatgc cgctcgggac ttcgttgtcc ctccaggcgc ccgccctggg gtcctccatc 1500

acccgccgtc acctgggcgc ggggaagctg gcgggagggg aggcggggct tggcggcagc 1560

ggcgggtggg ggccggggag cgggggcagg ggcgggcgga cggagcgcgg ggctggggac 1620

ccggggtccc agaagggggc gcggggacgg ggtccgagga gagggggccg gggcggggca 1680

gggcggacag ggctgggggc ggaggtccgg gggtgggtcc ggcggcgagt ccgggtcggg 1740

gcggacagag cagggggcgg gggtccggga ggagggggcc ggggtccggg aggagggggc 1800

ggggcccggg ggcggggccg gggtcggcgc cctgcgggga ggccggccac gtgacgcccg 1860

cggcccggcg gggctgccag gcggcgagcg ccgcggcggc cccgggaggt ggcggcgggc 1920

gcgagagcct gggccgcgcg ggactgaccg tcggggcccc gggacggcgg ccccggggcg 1980

cccatgccat ggagaagctg g 2001

<210> 7
<211> 3044
<212> DNA
<213> Homo sapiens

<400> 7
gcgcgcgtgt ggaaggctgg gtggggtgcg cacacacgct cacttgtgta cgtacacaca 60

cacacacaca cacacacaca caggcacaca cacccgagct cagtcaccca cattgcttgc 120

ccagggcgcc tggaagagct ggcgagcccg cccagctctg ttcacccggc cccgccccgc 180

cccctccggg agggctctcc gggcggcgcc cagccccgag cagagcaaag gacggcggcg 240

gccacctccg ttctccggcc gctggtttct cgtctttcct cgtcctttca ttgaacccat 300

ctccgtgctt cgaaaatctg actctaaccc gatctcttcg cgtctccgcc tcttctttct 360

agagtgagcg ccaaaaaggg cctgacagac agaagccttt ggccccagca ccggcccagc 420

ccgtctctag acgattcttg ctcctttcac cctcacagcc tccagtggtc gcttcatctt 480

cgcaccctcc cggccaaccc taactctcct cgtctctcct cgcgctgtct cgcgtcctcc 540

cctcaggatc cttccgcaca ttctcagcgt ccagcgcggt ttcccacaac ttcctcacgc 600

cccgctcccc tcgccctgtc cccgccctcg acaccacctg cgttccccac tcgctccaac 660

ctccctcccc cgctatcccc acttgtgggc ctccagctct ctgccccttt cctggccccc 720

atccctgaca ccccagggac ccttcctccc tcctcacgtt ctccctcctt ccaggatccc 780

gccccgacac ttcggggccc tcccgctacg cgcactcttt ctcctcaggt cctgacacct 840

gggcgccccc tccctgtcac ccaccttcag ctccagccct gactctcggg cgccttgcca 900

cccttacgct ccccgccccg ccccggtccc tcgggcgccc ccactcgccg cctctacctc 960

cctacctgct acacctggca cccctgcccc cacccctgct catactcttc ccccgccccc 1020

gacacctcgg gcgccccctc acacgctcct attctccaca cttccgtccc cgaaaactcg 1080

ggcgccctct cccttcacgc tcagtctctc ctccctcccc gcccggcccc ggacccagcc 1140

tggagatcgc gctcgggagg ggcggctgcc gcccgcgggg cgcccgcggt gcccgggcct 1200

gggcagcgag gaggtgacgc cgcccccgcg ggatgagccc gggaggcgga ggggcggagg 1260

aggtgctggc ggcggcactg agcggcggcg gcgcagggcg cgcgggcctt ccgcgccgac 1320

tccatcgacc caaggggcgg cggcggtggc ggcggctgag cgaccctggg ccgggcgcgt 1380

gatgaggagg ggccggcgcc agaccccgct gcacgtcgga gctcgcctgg atccgggcgt 1440

tggcagccga agggccctgg ccccgggact ctccgccgct agcccccgtc atatcttctc 1500

cgctttcgct tctccactct agccgggggt ggggtgggtg gggttggggt ctccgcgggg 1560

gtttccggcc ccgcggcccg ctcccgggtg tgcctggagg agttctccct ctgtggcgcg 1620

cgggagccct gtgatgcgtc agccggcggg acggatgagt tgcttctccg ggaaaccgtc 1680

ctcgcttcct cacgaccctc tcggctcccg cctgggtgcc cctcgggccg gcagtactcc 1740

gcctccgggc gctcgaagcg agttccccgg gggcttgttc gcaggcaccc cttcccctcc 1800

gaggcggcgc gcgcgctccc ggccctgacc gcggccggac acactcgcgc cccggtccgc 1860

ctgtcgccct cccgcctgct ccctccagtc accccaccct tagctgtccc cgccacctta 1920

ctccaccacc ctcccccgcc tctccgcgca ctccgcgtcc cggcctccag ttcccctttc 1980

ccttgaaccg ctcacttcac agcccttcgc ccccgggaag aagaaacatt tcccgaagcg 2040

cactcctcag ccctccttcc ccacgcgctc gccctcccct ccccctgctt ttcttggggg 2100

aggggggctg tcgccttgga ttgaaggcca ttgatttgta tgtatttgtc ccagcgctgg 2160

aggctgcccc agccgccgcg ccggtgccgc cgctgccagt ggagttgcct ccccgcttcc 2220

ctagggtggt tcggctccac caaacatgtc ggctcctgtc gggccccggg gccgcctggc 2280

tcccatcccg gcggcctctc agccgcctct gcagcccgag atgcctgacc tcagccacct 2340

cacggaggag gagaggaaaa tcatcctggc cgtcatggat aggcagaaga aagaagagga 2400

gaaggagcag tccgtgctca agtaaggacc tggctccata ttcccgcctc tctccctgcc 2460

ctccgccccc tcgcccactg ccctgcggcc gcctgcgcgc cccagttcgc cgccctccct 2520

cccgctggcg gcgcccaggc cacgagggct gcggccagcg ccggccgccc gggctgtttt 2580

aggggtgtct gagagcaggg gtgtgtgtcg ggagggaggg gcgccaaggc cggctgaggt 2640

gagggtggcg agccttaggc ggtgtgattt tccttggcgc ctttccggat ttcctcgctg 2700

gtcatcttgg ctccggggcc ccagcgggac tggggctgaa cccaggctct gtgcgtaccc 2760

tctcctttcc cgccgcgctg aggcagtgac tggggcacag aatccaatat ggccgtgcac 2820

aggtgctccc tggacggacc cggcgaaggc gcgctggca ggggatgcgg acgccaccct 2880

ggtcccacgc ctccgcgggg cggctctacc agcaacgcgg gacagagcag ggctgcccac 2940

agtggctgcg agcagcgggc ggcggcgcgg gctaggggcc taagctctgt cgcggtcggg 3000

tgggtgtgcg tccgccgcca tcttccagcc cctccccctc ggcg 3044

<210> 8
<211> 801
<212> DNA
<213> Homo sapiens

<400> 8
ccgcggtgct acaggtttct ggggccttct tcccggcagg gccacgccgg tttccaacgc 60

ggggggcatt tttcggcctt cccacggttc ccgctgttcc cacgaagaca gtgtctgcgg 120

ccaggcgctc cgagagagat gcggccttcc ccgggccggg cctggccgcg gcctgcccgt 180

ggtcccccgc agctcgggcc cgcagcgcga ggccacagtc cagggggagc cggcaggcgg 240

cctcctcccc gagccggagg agctgcgcgg acgcagcggc ttccaggcca ccccaccccg 300

cgccagcctg cacctgtgcc gcctgggtgt cttccccgag actctggtac tgtgaagggt 360

ccgggtcgcg cggggcgtcg tccggagcag ggcggactcg ggctttggcg cggcctttgc 420

cccggttttt ggcgcgggag gactttcgac cccgacttcg gccgctcatg gtggcggcgg 480

aggcagcttc aaagacacgc tgtgaccctg cggctcctga cgccagctct cggtcgggac 540

cgagcgggtc tctccacggc aaccgccgac gtcacgaacg tacaactgta ccgtcgcgag 600

aggacgtgat gcgcccggtg attggcgccg ccgctgcggc tgcgcaggag acgacccccg 660

cgggcgctcc cacccccatc tcgcgcggac tcgctttagg tctcggcgag tttctctgat 720

atgcgctcgc gggggtgctg ccatttcatc tcttccgcgc gggctcatcg tgctctcagg 780

gtctcgttga acaaggcaac g 801

<210> 9
<211> 314
<212> DNA
<213> Homo sapiens

<400> 9
tcggccgccc gagggagttt cttttattcc cagttcggct ttcttttgcg aaggccgaga 60

tctgggcctg ccaggggcct gcccgagtcc tctatcgcgg gtccacgtgg ccaccaatga 120

cccgcggcgc ccccgcgtgt ccccgcagcc actccgcgga agcagcggcg ggagcgcacc 180

accttcacgc gttcacagct ggacgtgctc gaggcgctct tcgccaagac tcgctaccct 240

gacatcttca tgcgggagga ggtggcgctc aagatcaacc tgccggagtc tagagtccag 300

gtgcgcactc cccg 314

<210> 10
<211> 2213
<212> DNA
<213> Homo sapiens

<400> 10
ccggctttaa acgcctctcc agccacctgt gaaccgcgaa ggagccggct ttcgcggcgg 60

ggaccttgcc accagtaccc tcgcgggccg aggtcgttct cccggtcggc ttcccgcctc 120

acccgaaaag gaattagagc atctacccaa gacggtgact ggcagggcag atcaaggtgt 180

cctggtctcg gccccagccc cgcggtgcgc cccgcccgct taccttgacc gggtgcaggt 240

agccatcgcc gcgcagggcg cccaacccgg cgtccgccgg cgcctcggcg tcgtcctgca 300

ggctgcgggt gagatgcgcg atgtaggtgg tggccagcag cagcacgtcc agcttggaca 360

gcttggtgtc gggcggcacg gacggcagcg tgcgctgcag ctccaggaaa gcgtgccgca 420

gggtctgcac ccggctgcgc tcccgcgccg cattcgccgc cgccggccgc ccgctcccgg 480

aacgcgagcc gcccccaggg cccgccggcc ccggcccggt ccgcccggga cgcgagtcgc 540

ggatggcggc ggccaggggc gcgggctcgg cgctggcgct gagggggctg cccgctgggc 600

ggccgcggtc catggcagct tcccgcgccg cgcgcgctgc aaaggaccga aggtgcggtg 660

aggccggggg gcggtcgggc ttaacccgag aggcgcagcc ccctggttct ccccgtgcgc 720

ccaccagcag cccaacgggg ctaagggcgc tctcaagcga gctcgttttg cctgggacgc 780

gatttgcttc cggacgtctg gggagagttg cggaactccg gagttcttgg gcttcctaga 840

aggataagaa gaggcgcagt gccggctttg cttttcaggg gcaaattaag caaaaggtct 900

actctacccg ggaagaaaga tctcggaagc acagctcagg atcagcactc gttcgcgctt 960

gggtgacttt atccaacccg gcacgcacga gaggtggcgc ggctccttct cgccgacgcc 1020

gcggaaaacc acggctcacc agccgccctc ggcctttcac gccagggggg atttctgccc 1080

gaggagcggg ggacccttag cctcacctcg gggtacggca cccgccaccg ttccgagccc 1140

gagagctgcg cagtacgcgt ctgacgggcc cctcaccttt cctggagcgg ctgagtggag 1200

ctccgctccg tcgtgagggc gggcgagggg cgtggagcag ggcctgtgtg gccagggccg 1260

cgctggtcac tccatcctcg tccggccgat gcccaagtcg acggctgttt ccaacctccg 1320

ctggctgtga cttttatgcg ggcgccccgc ggccaggcgt gtgtgctccg accggctaag 1380

gcaggtcggg cggaggacct ggcccaccgg agaggctacg ccgggggctg aggcggctta 1440

gagggtcatt aatcaaaccc tccggcgggg cgggctcggg ggcggggcgt cctcctggcc 1500

ccgcccctcg gctcactgcc tcacgctgct ttccccgagg cgcctcgctg agggcggcgt 1560

gtggagagtt tggggtgtct gccgccggct gcggtggggc cgggctggag gccgcgggtg 1620

aggcctgtgg ttaacctcgc gctgccgagg tcttacctcc tcgagtccag tctgattcca 1680

ggccgcttcc aggccggtgc ccagctgagg cgggaacgct gcagtttggt tgagcgtgac 1740

ttttaggctc tgtgaggaaa agtcgagcgc gccacatcga ggcgctagcc gtttattcta 1800

ccacaaggta aaagattcat gctgtcctag ttaccctaaa gctgggagat acactgcact 1860

tcctaccaga ccccgaatgc tctcagtgtc tgtaattctt taagaagttc ctagagcaga 1920

cagcccttgg atcgtgggca cttctccccg gggacgggga ccctgctgac cgcctccgct 1980

gcccccgcgg gggccaccgc tctttaatta tttgggcgaa acattctttt ctggttttgc 2040

acttgtggac tcacgggaag cgtgacttgc agcgaggcag gacccgatcc caggcttctt 2100

tagaaagcgg acgctgcgcc ccaaggcctg ttcagagccg ccccaggaag ccgtgggtcc 2160

ccgaccgccc caaaccgcag cggtttctgc aggtcctgga cccgtcgcct tcg 2213

<210> 11
<211> 714
<212> DNA
<213> Homo sapiens

<400>    11
gcggacgcag tcacgagtcc agggcgaagc aggcagggag gcaggtgggc ctcggtccgc    60

cgcaagctca cacttaggag gaccacgggc cgcatgctgt cgtcgtcaag gcaacgacct    120

cactctgtcc ccaaccatag gcacaaagtc ttgggagaca gatacggccc aggtcagaat    180

gcgttcacgg caggcaccaa cacctgtgaa ggccaagggc tagagagcaa ttagctgggt    240

gagaggcacc acctcccagc tcgtaaggcg cccagtacct ggagcctggg aacctgcacc    300

gctccaacta cccctgggcg aaggcgttgg ccgcggagct gcaagggggg gcggtttctc    360

acccgccccg agagcgccag gcctcccttc ttctgattgg ccgagccgag tcgtcacgag    420

ccatgattgg ctcagggcca accaccccgc cccttcacct agggctcggc ccaggttctg    480

ctccctgaca cgcagaggcc ctgcgtcccc acacgccttg gttctcgtca ggaggcgcct    540

ttctgccttc cccagcggga ggaggcgatt gtgatgccca cgcgaagggt aaaggtggcg    600

gttatgtagg actgcgaaga ctatgcaaaa tgcgatacgg tttccctcat agcatcgccg    660

ctggggcagg ggcgggcgcc gggcgccctg agtcgcgtag gcgcggcctg accg    714

<210>    12
<211>    8078
<212>    DNA
<213>    Homo sapiens

<400>    12
gcgggcaggc ccaagctgcg atgtggagaa ttcgatgtcc gagcgacctc ctcggaggag    60

tgggtcgagt taaatataac cgcgcgaatg gaatggcgct aaaaataagg cagcagctgg    120

cctgtccaca gccctgtccc gggagggcgg ggggccccag tggtcttggg caggaaggcc    180

gcgtccggcc caggggcgag aaggctgcgg cgtccgcagc cagggctgga aggcctggga    240

ggccgcgctc tgtgggcccc ggggcctcca ttcgggctgg gtcgcgggcc tggacgggga    300

ctgtccagag gcatccgaaa gccaggccaa cttgcctgga cgtaacaaga cggaagggct    360

gggcgctgag gtcctgccag cccggccgcc agagggagct gagcgccaga ggaggacaag    420

ccgaaccctt caggaggccg ggcgtctccg gagaccgaag cgccggagga cccgaggagg    480

tctgccccgc gcgctgctct ggagactccc ggggcgggtg gcgctcggcc tttccgctcc    540

cttccttccc acaagtccct tcccgcgcgc gccccacggc cctgcccgcc ctcccgcgtc    600

agcgccccaa ccgtcaagcc agcaattgaa acgtttccaa aacggtctat ttatttgctc    660

ccaataaatc gatcggcggt gattaaagaa tcgatgtggc ctgggtgggc gagtcgcttg    720

aggggaggga ttgggggctt tcgcccggcg cctgcaggga ggccgagggc gggcgcgggc    780

ctgaggaggc cgtgtcccgc ccgggccaca cccgaggacc cgacacctgg gctggcaggc    840

cccggcaggc agcgttccct ccggcggaga ggggcgcgcg cccgccgcct gctttcctcg    900

gcccctctcg cctttctcgc gcgccgggga ggctgtggcc gccagtggct gcggagctgc 960

tcagaggctt ttgttgctcc tcggccggct gaatggggat tttgtaaagc gggacagata 1020

aaaatgagca gcatcatatt gtttgacaga atgatctcgc atgatgaagt gtcggctccg 1080

aagggggtga aaatggtgaa ttcctaaaaa cccagccctg ggctcctcct cgagctgccg 1140

gtagcctgga gggacccagc ggacagccgg gcctggccgc atcgctccaa acggtgtcag 1200

aaagactccg gctttcaatg ccaagtcatt tttaagcccc gatcctgtcc aggacctttc 1260

tcctcgtgga tgaaaagaac aattttcgag agaaaggctc gtttttatta aatccgacat 1320

gctgctgata actccatgct aatgtgaaat aattaacata atagccataa ttaaaagcac 1380

gctaacaatg ccataaattt atcacacaat tttactagct ttctgcccct aactgctctc 1440

tcatcgttaa ttaaacgtgt tgccttttac agaatggatg tttatatatt tccaatataa 1500

ataaattcga aaccatcctc tctctcttcc tctttctctc ctcctttcct tttggtctct 1560

cgccatttac aggcacgcct tggcgtggac cctgagtggc agacatcttg aaaataaatg 1620

aagttttgag atgcaaatcc aaacaagaac attaaaatag cctctttttt tccaccccga 1680

aaagatccgg agaggtatac aagggggtag tggtgggtaa gagagttgaa aatcccccgc 1740

tttgggaaat ggaagtaatc tgggtgggtt ggggccttgg gtaccacctc tgccctttcc 1800

caccttcctt ggtggcggcc atccagacaa agaggccggt aatagtttaa caaatctatg 1860

aagattttca agaagcagca gactttgatt gttgcgggcg cgggggtgtt ggggagaaag 1920

gaggggaatt tttctaatag tcccacccac gttttgctcc ctcttggaca aagagtaact 1980

actcttggtg ggggacgcgc ccttcactcc gcggaacctg gtcccaactc cccgtattgt 2040

aagaaaagtg cacccgcgcg cgggcatgat gattctatct cacatcgcgc caacgactta 2100

ttcaagccac tggcactgtc tctgacttaa aagaggagaa aagaggcata tgggttcact 2160

tgggcctggt gaggggtagg tgggcaattc ccgccttccg cactctaacc gtgcccctcc 2220

tccagtgttg accacctaag aacccaaaat gagctgtaat taatttccct ttctccatca 2280

taaatttttc tatccatttc ttccccccca tccccccact ggacgcacac actaaatctc 2340

ccctcccctg gagacgtctc aatttccttc ctatcgatcc ggactccatt cttcttgcct 2400

cctgttgcta gaacctagat ccccactccc cgcacccctc attcccaccg cgtccaggtg 2460

gctttcccag cggggtacca tgtactctgc ccgctccaga ggaaccgaag gggtttcatt 2520

ccattctcct ttggttgaaa catttcaaac atttgagcag gtgaggcagc tggctgccat 2580

cttccttttt aaatctctcc tgggaagttc gcttgttgag actcaaagag tcactcaaac 2640

tcataattgc gtgtgtgtgt ctactcattc tccctctatc tctccaataa ccctttgaga 2700

ctcagaaact ttttatccac atacaccctt tatcacattt tcttcccccc actacatgtg 2760

tctcactttc tctctgtatc tgtctcgctt cttccgtctc tgtcctacag cttggcggta 2820

actgacgacc tgtgagcttt tagctgcaaa ctgcaactac gcggcaaaca atttatttag 2880

cccgacatct agccggtctc cggcaggacc ctgcaccgcg tcgggatcgg acccttccgc 2940

tggggcggcc tcctgcgtca aggccagcag gaaccttcct gtcgccctcc ccggccgccg 3000

cttcgcctcc ttcccgcccc cggaggttgt gcaggcgcta tggtccgcct ggagggagaa 3060

agccggcggc cggttcctga gccgagagcg gccgcggaaa aatcctctgc ctccgctgga 3120

aatcgatatt aggccggcgc gggcgcggga cgtcggggcc gcagccagta ggttgtgcac 3180

gtctcatcat ttagctaatc gagtcgaaaa gtttctgtaa gggccggacc cagcatcaga 3240

tggtaacact gattgaacaa gagattagca caatagatct ctaaccgagg ggaagcgttg 3300

cttttcacgc tacgcgccgt aattaatggt atgaatcaat taatttgact tttattgtgt 3360

cgaaggaaaa aagcgcaaca aatggaaccg gcagctggga gttgttcgtc ctccaccccc 3420

ttccccaggg aggttccaag gagacaccgg ggaatggacg gatcaggctg ggccgtggca 3480

gagggagggt aggaggcagc gaccagcagc gtggagggag tccagagagc tagcctctgc 3540

ggacggcgga atcgaaatta ggctcatttg gagactactt cgagaccggt gaggggagcc 3600

ctgtagccac catcctccgg cgcgcatcca cacatactag tccacgcggg cccagccacc 3660

aaggccgcgg cagggccagc gctgcgcccc gggcccctgc ctttagggct gggcaaccca 3720

agcagagcaa aggaggttcc tgaatgtgta aatttccgct ttttagcttt tttttttttt 3780

ttttttggac cttccgacac ttcggttgct gaggcagttg cagacgcgac ctctgcagtc 3840

ctgggcgatg gccagccagc tcagctcggg tcggtttcgc ggaaagctgt ctagacggca 3900

ttgtaaacgg ttcggagcct gcgggccaca aagctgtgga gctacggaaa tcaactctga 3960

gatgcgtttt agggccgtgt gcaacctcgg gatcatttag ataaagaaaa actgtggagg 4020

ttggcgggcg tctcaggata gtgtcaccac cccctaccct gctcccagcc tcagatgagt 4080

agtgttatat cctgggaaac tgtctaatgg ggatgaaagt caatctgtgt gtctcaatgc 4140

ctgtaatgaa gcaagtttac agatttttaa atttttattt ttattttatt gaattatttt 4200

tggtgtgtct aggccaagga aagaggagat cgtgggtggg gaaacagact gagggaatca 4260

gaagcaccac tgtccatccg gaattaaatc cacatcccag catcttctgc aaatatttca 4320

ctaattattt cctctcggaa ctcctcccct cgtgctcctt cctctggtga ggccggcgct 4380

cccctcccag gccgcagcgg acagacaggg attgggttcc gtgtgcctgc cacaccaggc 4440

aggctcttgc ggctcccaac taggcggcct aaatgaggga ggaaagagga ggcgcatcgc 4500

tgattcaccg cgtcaagagc actgactttc cttggaggtg tgaggtccac gcaccccagc 4560

cacgcacttg ggggtcggtt tgcggtgcct ccccctccag tcccagtgaa atccccacag 4620

tttttcctac tatcactgac ttgccttgca ctccgcgtgc attggccaca catcctcgcc 4680

tcctccaccc gctccgccgc cggttttctt ggaagttaaa tcttggagga tttgtccaca 4740

ccttaagaga agaaaatcca cgttagctgg cagcaacgga gatcccagca tgctggcatg 4800

cccaagtctg cccaggttcc cccaaggcca tgcccgccgc ccgggaagtc actgcccgca 4860

cccctcacgt ttcttcagcc gcccctgggc gctgcgtcta acctgaagac accaggcctc 4920

ttcccggatc cactcgactt acccaggccg ctgccaatcc cagctccttc cccagcgcct 4980

catttccgat tttttcatat gctaagtcgt ttaacaactc caagtagcca gttatggctt 5040

ctttatttat aggttccctg ctattttacg tcgtttttat ttctctcggc aactattcta 5100

gtagattaat caatagccat tttctgacct tcgggaaccc cagctgatgc tttttgtggc 5160

cgcacgaaaa aatacataca ggaaaacacg cccgcatcaa gccgggaaag agcaggtagg 5220

acctgagtgg tttggttggg ggagggggaa aaagacatct cagcaggtgt cttccccgga 5280

atgagcactg aggccagagg ggaatctgaa atctaattag caggagggag ccgggtgcgc 5340

tgctcttact ctttaaagct aaaaacaatg aaacaaaaag caaaacagag actaagtttt 5400

gctttttaaa acacgatatg ggaacctcgt tctaggtcgc ccagtccctg tctaaggagt 5460

gtgacaaagt gggggggaga agggcggaag ggagaggggg cggggaaggc agggcagcga 5520

cagtcgcaca gtcccgcgga cgctcccagg cccacgccct gactcgctca cacccaccca 5580

cactcacacc cacccgctcc ctgggcccca gggcccggat ccagcctggg tgggggggtc 5640

tccgggcggg ccgcagcgcc ctccgtgccc cggggatgct ggcgcacagt gcggagcgga 5700

gttgcgcgtc tctcgtccct ttgttgacaa ttccctgaac caacttgagt ttggccggct 5760

cggccgcggc cctgacgtca cgcacggtca cgtggccccg cctcccgctg gatctttaag 5820

tagaaagtaa tctatcaggc cagtccttaa aacgggactt tcgactaccg gggcttcggc 5880

gtccctgaca cccagccccc tgcccccccg ctactgtccc tgcccgcgcc ctcccgagct 5940

gctcggcgcc cggcgtcccg cgcccgcctg gaccgctcct gcgccccacg ccagggccag 6000

aggccgagga aggcgggcta agtgaggggg cgcggcgtgg agaaccgccg gggccgggag 6060

cggtagcgag cgcctagtac cgagcgccag ggacggcagg agttcgcgga gcgcggccgc 6120

tgggggcgga cggcagagcc cgcgccacgc gatgcggggc cgccgagtgt gagctgagcc 6180

cagcgggccc caagccacct gcggccccct cccctctccc tgccccccat ctttcggggg 6240

cactcaaacc ctcttcccct gagctccgtg gcagcccccg aacaccctca tcgcccgctg 6300

ccccctcccc gccgccgcta ccaaccccga ggagggatga ccctctccgg cggcggcagc 6360

gccagcgaca tgtccggcca gacggtgctg acggccgagg acgtggacat cgatgtggtg 6420

ggcgagggcg acgacgggct ggaagagaag gacagcgacg caggttgcga tagccccgcg 6480

gggccgccgg agctgcgcct ggacgaggcg gacgaggtgc ccccggcggc accccatcac 6540

ggacagcctc agccgcccca ccagcagccc ctgacattgc ccaaggaggc ggccggagcc 6600

ggggccggac cggggggcga cgtgggcgcg ccggaggcgg acggctgcaa gggcggtgtt 6660

ggcggcgagg agggcggcgc gagcggcggc gggcctggcg cgggcagcgg ttcggcggga 6720

ggcctggccc cgagcaagcc caagaacagc ctagtgaagc cgccttactc gtacatcgcg 6780

ctcatcacca tggccatcct gcagagcccg cagaagaagc tgaccctgag cggcatctgc 6840

gagttcatca gcaaccgctt cccctactac agggagaagt tccccgcctg gcagaacagc 6900

atccgccaca acctctcact caacgactgc ttcgtcaaga tcccccgcga gccgggcaac 6960

ccgggcaagg gcaactactg gaccctggac ccgcagtccg aggacatgtt cgacaacggc 7020

agcttcctgc ggcgccggaa acgcttcaag cgccaccagc aggagcacct gcgcgagcag 7080

acggcgctca tgatgcagag cttcggcgct tacagcctgg cggcggcggc cggcgccgcg 7140

ggaccctacg gccgccccta cggcctgcac cctgcggcgg cggccggtgc ctattcgcac 7200

ccggcagcgg cggcggccgc ggctgctgcg gcggcgctcc agtacccgta cgcgctgccg 7260

ccggtggcac cggtgctgcc tcccgctgtg ccgctgctgc cctcgggcga gctgggccgc 7320

aaagcggccg ccttcggctc acagctcggc ccgggcctgc agctgcagct caatagcctg 7380

ggcgccgccg cggccgctgc gggcacagcg ggcgccgcgg gcaccaccgc gtcgctcatc 7440

aagtccgagc caagcgcgcg gccgtcgttc agcatcgaga acatcatagg tgggggcccc 7500

gcggctcctg ggggctcggc ggtgggcgct ggggtcgccg gcggcactgg gggttcaggg 7560

ggcggcagca cggcgcagtc gtttctgcgg ccacccggga ccgtgcagtc ggcagcgctc 7620

atggccaccc accaaccgct gtcgctgagc cggacgactg ccaccatcgc gcccattctt 7680

agcgtgccac tctccggaca gtttctgcag cccgcagcct cggccgccgc cgctgctgcg 7740

gccgccgctc aagccaaatg gccggcgcaa tagggacgcg ccaatggccg ggacccaggg 7800

tccggcggcg gcctcgagca acaaatgcac ctccaggctg cgcgccctgt cccaagcccg 7860

gtcccggtcc cgctgcccaa tcctggactc tgcctctccc caatttcctt tcccctgagc 7920

ccccaacgcc taccttccgc ggcctccatc ccctcgcgca cacctaagct ggtcgagcaa 7980

actcaccgcg cgcccgccgg ggatagcttt ccatacaggt aaaaccgaaa accgaatttt 8040

ccaaaaatgc accccgacgg cgcctgctct tagtaccg 8078

<210> 13
<211> 495
<212> DNA
<213> Homo sapiens

<400> 13
ccgcgccctg gaccatccgg gcgtagtccc ggcagcaagg ccttctttcc ttgctagcct 60

gggcctgccg cagacagacc ccagagggag ccgcgcccag cccgctgggc ggccccggct 120

tcccgcgacc ccctccagac cctgggcaga aagagcgccc tgctgtcccg acagagccac 180

tgtgcttttg agggatcctg acacctagtg gctcccgctc ccttctccga agagcaccgg 240

gtcctatctg agcattcccg cgactcccag cccctgatcg cagctaagac acccattcgc 300

gcacccggct tctcccacat cctcgtccca ggggttcagc tgacactggt agtcgcctga 360

gctgtactct ttgggcccca ggcgccttgg cgggagctca ccctccctgt ctccccagct 420

gaccctgccg cgcccccttc atctccgcac gctcccaccc ggccccctcc acaggctgtc 480

cagccccgcc cctcg 495

<210> 14
<211> 1192
<212> DNA
<213> Homo sapiens

<400> 14
tcgggcctcc gctcgacgga ctgccttgtc cactctccgc ctgggaacgg gggttcgtgg 60

gagcgcctta gtggaagttt gtggagctcg ggaggtggca tgcacaggcg cctcggagcg 120

cggccccgag gggcgccggc aggcgagagg cctgcactaa ccggccgtaa gcacagctct 180

tttgtactct gttttccccc taaagacatc tgatgccccc agtgaagaaa agccaacagc 240

agcaaagcct gatggagagc atgcagcccg ggaagcccag tgactgggag ctggagggca 300

ggaagcacga gcggcccgag agccttctgg caccgacgca gttctgcgcg gccgagcagg 360

acgtgaaggc gctggccggg cccctgcagg ccatcccgga gatggacttc gagtcctctc 420

cggcggagcc gctgggcaac gtggagcgct ccctgcgcgc cccggccgag ctcctgcccg 480

atgcccgcgg cttcgtgccc gcggcctacg aagagttcga gtacggcggc gagatcttcg 540

cgctgcccgc gccctacgac gaggagccgt tccaggctcc ggccctcttc gagaactgct 600

cgcctgcctc ctccgagtcc agcctggaca tctgcttcct gcggcccgtc agcttcgcca 660

tggaggccga gcggccggag cacccgctgc agccgctgcc caagagcgct acgtcgccgg 720

cgggcagcag cagcgcctac aaactggagg cggcggcgca ggcgcacggc aaggccaagc 780

cgctgagccg ctctctcaaa gagttcccgc gtgcgccgcc agccgacggc gtggccccac 840

gcctctacag cacgcgcagc agcagcggcg gccgcgcgcc catcaaggcc gagcgcgccg 900

cgcaggcgca cggcccggcc gccgccgccg tcgccgcccg cggcgcatcc aggaccttct 960

tcccccaaca gaggtcccaa agcgaaaaac agacctattt ggaagtaagg agggtaaagt 1020

aaaaccgaac cgaaacccac agcgtcgacg gccccaggcc tagatctgca ggaagcatcc 1080

cgagttctcc tagcgtggag aggagcgggg ccgggccagg ctagggggcg gctgcgcgag 1140

ccgtcggcgg gtggaggcgg agggagagca ggggcagccc ccgcgccctg cg 1192

<210> 15
<211> 1001

<212>    DNA
<213>    Homo sapiens

<400>    15
cctgggaaga gctgctgggt ggggctgtgg ctgccagagt ctttcccaaa ctagcacaga      60

acctgttttg caaccctggc agggtggagg caggatccag gccaagagct ggtcagcagc     120

tgaccccgcc cctgcctgac ccctgcccct tccactgccg aagagcccct ggcaaatagt     180

gtaactcaga tcgtagaggg tgcagattgc tagaactcag ttccagaagg tttctccacg     240

ataatgtcat gacttaagta cacagttttt ccatttttgt ttcgtaactt gattttttaa     300

agcagtcgct acagaacaga atctagacct gtattttata gcatagctgc ttgcatgtat     360

ttttcaagac ttttctttcc ctcagagtga tgtttgggtt ttgttttttt cctgggaagt     420

tggtgggggt gggagctaca tagcccacct ctttccccag taagattctg gtccctagga     480

agaggggaaa acagctcggg cggctctgaa gaggaaatct caggccctag atgctacagg     540

tcattgttag caaccccagc cgctcccagg aaaccagcca gcagcagcgg agggcagggc     600

tgggcgggca cagggtcccg actataccca gtttgcagtt cggcccaccc ccagcaccag     660

gaatgcccct cccaggctat cgctcctctg caggcttccg cagctcccca gcccctgtgc     720

tcctggagcc tgcctgcctc ctgcccgcct gcgtgactca ctgagggccc cctccctatc     780

tttcactttc acccagcacc cagaagggga gtaatttcct cctccatttc cttcctcagc     840

tcctgggcct tgaagagagt aagagaccct cctcgtgtgc agcctttgtc ttttcatata     900

tgaagctgga gggagggaga ggcacagaga ctaggagggc atccaagtca ccctcacccc     960

cagcaagagg ggagtggggg gatttggaca agaagtgcag a                        1001


<210>    16
<211>    2001
<212>    DNA
<213>    Homo sapiens

<400>    16
taaatgagtg aatgaatgaa aattatttta tttttatttg agctttggtt ctgccatttg      60

ctagcagtgt gactcaagag aagccagtaa cccccctgag cttccctagt tcacaaaatg     120

cttgtcatga agtcgacagc ttccggaggc tgcgaggctc gcaagaaatg cccacatgaa     180

tgtgcgctta gggcgtgagt gctcactcca gaaaactcca acacagtgaa aaggcagaag     240

cggtgttttt cttttttaca tttttataag aatatataaa aaatgatata aatggacatt     300

tacggtagtg ggggaaggca tatatctacg ttaaaaggca ggacattttt aaaagctcta     360

ttttctaaat gaaaactacg aaagcggggt gggttgtggc gggggcagtt gtggccctgt     420

aggaccttcg gtgactgatg atctaagttt cccgaggttt ctcagagcct ctctggttct     480

ttcaatcggg gatgtctgca gagggcagaa agaaaacagg cgttagaaac ctgaggtcaa     540

agatgtgtgg cacatcccgc cctcctctct tgccgtccct accggcattg aaatacttat 600

ggataaagtt ctcgcaatgg cttcacgtgc atgtacccgc cgccaccgct ctcccacacc 660

tccctggtcc agcagctagt ccactgcccg cctggctgct ccaggcgcgc cgaccgctca 720

agcgctccag gtccacccgg cggagggcag agaaagcgcg accgcgcggc ccgcagggtt 780

gcaagaagaa aacgagtgtt atataatgag tctcagtggt tgctcacaat gccaggcgcg 840

aaggcgtgaa gatgtggcct ttcccttccc gcatccccag gcatcttttg cacctggtgc 900

ggagtgagcc agccagcttg cgataaccaa agggcgcctc aggctctggc gctcctcggc 960

ggaatcccgt agcttcccta cgcatgcctg cttctacaaa cccacaaatg gtttccgatc 1020

atttctgaaa caaaatggat gctcatttat tcatgtgctc tggcttctgc cttcctctct 1080

aatctcgttg cgtatgggct ccagctcgcc gttcggttct cccgaggcag catttacact 1140

tgagagtctc aagattattt tattcctgag ggagcatttg cacttgaaag tctcttttta 1200

cgtttattcc tgaggcagca tttgcacttg agtttctttc tcccgtagct tgcattagat 1260

tctccgacca ctctttagct tctcctccta ttcacacttc atatttaccc attgcattgg 1320

ttttataaac tcgctctctg aaaatagatt gttatcttcc ttaacgtctg tttcccaggt 1380

cgggcaagat agcttgggac tgtaatccca gtactttagg aggaggaggg gggatgatcg 1440

cttgagccca gataacatgg tgagaccttc gtctctatta aacaaacaaa caaacccagg 1500

cgtcgtggcg tgcacctgtg gtcccagcta gtcgggaggc tcaggtggga gaaccccttg 1560

agccagggag tttgaggctg cagtgagctg tgatcgcgcc actgcactcc aggttgggca 1620

acagatcgac tctgtctcca aatgtaaacc ccatgagggc aagactcttg tttggtctca 1680

ttcaccttgg cgtgcccacc acctagaaca gggctgatca cgcagtagaa tctaaccata 1740

taattaattg tgcttgaaga gggggtgttg gggagtaaga gaaggaaggg aggagggaag 1800

aaatgaaaga cttgtgtgtt tggattaaat atattaggtt tggttaagag tcgttcagtt 1860

tattcatttg cttgtggccc aattcagtag ttttactccc tctcccactt ggctcctcag 1920

gctttttgct cagccctgga accgcgctgt aattggcagc tccttctaaa tcgggacccg 1980

gatgctagct gtaactggag c 2001

<210> 17
<211> 1834
<212> DNA
<213> Homo sapiens

<400> 17
tcgctccgga atggggaagc ggctgcgccc tggacggaga ggggcgggga cttcgcgact 60

gcaggcggag ggagggcggg tgtcgctggc gcaggcggtg acaggagacc accgccgcca 120

ctgagtattc ctatgcaagt ttcttcatct tcctgtgcat cagtgtttac actggggtaa 180

tgataaatgc tgtgttgaaa aattatttga tggggccatg gaaggaacgg aaggaacggc 240

gtcctggccc gctcggggcc cgcgcacgcc gccaccaagc cgcgggggcg ggtcggaggg 300

gagagttgcg tcagccaggc cgctgtcaga tgacgagccc ggggcgtgac ggggtggagc 360

atccccaaaa aagtgcatgc ctaggatccc gcccagtgta tccctgcgcg cggcgggccg 420

ggctgggcag ctttataaac agccgtggtg tgagcctcga agggaaccat cagcgcctcc 480

tgtccacgga gctccaggtc tacaatggca gcggccgcca gccccgcgtt ccttctgtgc 540

ctcccgcttc tgcacctgct gtctggctgg tcccgggcag gatgggtcgg tgagttcggg 600

gatgtagcct aagcagggcg ggggccaaac ctgggaggtt gtggactgca gcgggtttca 660

gaggagggga ggcttctgga aggaccggcg cgatctccct gaacgaacat cgcggtctcc 720

ccgaacgtcg cggtccctcc gaacgtcgcg gtctccccga acatcgcggt gcccccgaac 780

atcgctgtct ccccgaacat cgcgatctcc ccgaacatcg tgatctcccc agacatgccc 840

agctgaaggc actcagttcc cctcggtggc tcctttccgc cgggtccgct tcctgcggct 900

gctgcttgcc cctcaggcca ggaggtttct ggaaggaccg gtgctgtctc cccgaacatc 960

gtggtctccc cgaacatcgc ggcctctccg aacatcgccc tctctccgag caacgcgatc 1020

tccccgaaca tcgcggtctc cccgaaaatc gcgatctccc cgaacattgc catctcaccg 1080

aacatcgcga tctcgccgaa catgcccggc tgaaggcact cagttcccct ccgcggctcc 1140

tttccgccgg gtctgattcc tgcggctgct gcttgccccg caggccagga ggcttctggt 1200

agcaccggcg cgatgccccc gaacatcgcg ttctacccca acatcgcgat ccctccgaac 1260

atcgtgatcc cccccgaaca tcgccgtccc cccgagtaac gcggtctccc cgaacatcgc 1320

ggtccccccg aacatcgcgg tacccccgaa catcgccgtc tccccgtaca ttgcgatccc 1380

ccgaaacatt gcgatctccc cgaacatcgc gatctcgccg aacatgcccg gctgaaggca 1440

ctcagttccc ctccgcggct cctttcctcc gggtccgctt cctgcggctg ctgcttgccc 1500

cataggccag gaggcttctg ggtggaccag cgcgatctcc ccgaatatcg cggtctaccc 1560

gaacatcgcg gcctccccga acatcgcggt ctccccgaac atcgcgatcc cccagaacat 1620

cgcggcctcc ccgaacatcg cggtctcccc gaacatcgcg atcccccaga acatcgcggt 1680

ctacccgaac atcgcggcct ccccgaacat cgcggtctcc ccgaacatcg cgatccccca 1740

gaacatcgcg gtctccccga acatcgctgt ctccccgaac gtgcctggct gaaggcactc 1800

agttcccctc cggggctcct ttccgccgag tccg 1834

<210> 18
<211> 597
<212> DNA
<213> Homo sapiens

<400> 18

acgagtgcgt gcgcttgatc tggtttctgc tctctgggag gtgagtggcc gtgcggggcg 60

gtggcagctg gcgacacctg cgggctgttg ggcaccagcc cggggcgggc gctcgcacct 120

gtcgggcgtg cacaaaggcc cggcgcacgc tgtgggggcg gggcctcccg ggttggccaa 180

tgaaaagctg gcactgggtc ggaggcgcca gccaagtggg gggcggagct tccaccaccg 240

gccaatgggg atctggcttc gggatgtggg cggggtccac ccggtcgcaa cccgttgagt 300

ctctgcacag ctgccgcgct gacgcgtttt ccgcgtgtcc cgagccccgg cggccccgcg 360

agctcggtcc gtgcggggaa agcagggctg acgccgtctg cggagaggac tgcgcagccg 420

ggcttgtgtg gggccgcgcg taacggcagc ggctactccc tgcccaggcc ggccagcaca 480

gggccatggc cgaggcggct gcgcctccgg taagggcgac cctcatggag gcttggggac 540

gtggagccga gtcctgaatt cgccaggagg atgttccacc ccccaccatc tccggcg 597

<210> 19
<211> 481
<212> DNA
<213> Homo sapiens

<400> 19
tcggatcggc ctcccacgcg aagcttgctc cccaccagca tccccacgtt ggtggcgacg 60

ctgccccggc cccacggata cttccgcgcc tgtcagactc cctgatgaac tacccttccc 120

agagtaccgc gggagctcgg gctcctgagg gcgacggtcc tctgatggca gatgcgggag 180

aaactctggc gtcaggcggc cctcgcgtgg agcacacgaa gtcgtggctt attctggctt 240

cagtatgtgg ggtggagaag gcgatccacg cagctgcgtc tatttcctgt ggatcaatcg 300

caaaatacgt tctgtaagcc ccgcccccac tgcgtgcggg cggcttttgt ctccacggca 360

accgtcaact ctggaaacgc ctgtctttct ccatggcaac tgtctacgcc gcaggctgga 420

gctgcccatt accggagccc gtaagcagta tgggtgctgg acaaacagcg tgatcgggtc 480

g 481

<210> 20
<211> 1001
<212> DNA
<213> Homo sapiens

<400> 20
gtctgtgttc cttttcttaa ctgtaagaag aaggctctgg tttcttcagg ttataatttc 60

attaaaataa ttttattgtt ttctgacctg aaaaaattca gaatatgtat atctgcttga 120

tattttcttt tgggcatctt ggtgcaacac ttaaaatcta tttcattttg tagtttggga 180

gccataattg cagcttcacc aggcttggtt cttcttggcc cgggcccttc ccttcccttg 240

ctggttagta ccagccgagc tggtttgctt tttccctttt tggtactatt ctcctcctcc 300

ttcctccacg ttaccttctg ccacggcctc tcttcttttt ccctccattt tcaatttaca 360

cttacatttt ccctcctcct cctggccctc cctagttttt cccctcccct ggtttctagc 420

tcctttttgc tttctgtttg tgttactgag ggcagtgctc caattacctc atatttggag 480

agaggaagct gcagccaatc cggtttctgt ctgcttttag gtcaagtgat ttctgaactg 540

cagtgagatg ctttgaattt gtcttgttgc agctctgagc ctgtaagatg gctgtctgaa 600

tcggcagcgg ctggaagaga cagagagagg cggggaggga gggagaaaga attggaggga 660

ttgccggcat agtgcatgtt tttaaatgtg catcgaatcc gatgaggcca aggttgggat 720

ttctgtggga tcccaggact ggcttagctg cgtttttgct gagattagga gaggaaggaa 780

atgggaaatt cactgggctg ttttaaggag ccgaaagagt caatagctat tcctgagaag 840

gctcccatat ctcctaagaa aagggttcgg ttcaaaagga ggtggagagg gaagaaaatc 900

cctactccag aggcatctca ccaggaagaa acctcagaag gaactggagt cattgaagag 960

actgaaaccc taacgaagtt aacagagagt ctccaaaagg a 1001

<210> 21
<211> 206
<212> DNA
<213> Homo sapiens

<400> 21
acgccaccgg tcgaggacgg caggagaccc ccgagtgcag agaaagctca aaccggcagc 60

gaagtcggtc ctagccaagc tgaaaaaacg tctcggattt cgcggacagc ggcctagaca 120

cagcccgatc ttccagtcct agtgccctgg tcgagacggt tctatccttt tgcaaagaag 180

ccggaaagag ctgggtcccg ggggcg 206

<210> 22
<211> 2001
<212> DNA
<213> Homo sapiens

<400> 22
acccttgtag gccggatgcg gtggcttacg cctgtaatcc cagaactctg ggtggctgag 60

gcgggtggat cacctgaggt tgggagttca agactagcct gaccaacatg gagaaaccct 120

gtctctacta aataaataca aaattagccg ggcgtggtgg cgcatgcctg taatcccagc 180

tactcgggag gctgaggcag gagaattgct tgaagccggg aggcggaggt tgtggtgagc 240

caagctcgcg ccattgcact ctggcctggg caacaagaga gacactccat ctcaaaaaaa 300

aaaaaaaaaa atagaacaac ccttctaaat gtaatccaca gctcactcac cttagtccac 360

acaatgacca ccacattttg gatgtctcca ttctgaagca ctccccagat ttccagacct 420

gggtgttcag ccacctactt aatgcctact taatgtctct gaaacatctc aaactcttac 480

atgaccaaat aaagctcctg ttgtctccag tgaatattac tgttaatacc aacttctcca 540

tctcagttga agaaccatgg ggtcatcgct gaatcctgtt tcactccctc gctgtctaca 600

tcagaaaatt tagttgctcc ctttaaaaat ttgcatccag aatgcaacac atctcctaat 660

caatgactct ggtccattac cctggactgg ctgtagcttc cactctgatc ttctttcctc 720

ttccctcaac cccacagtct gctctccacg ctaacgggat ggaccctgtt aggactttgg 780

taagatcacc tccctcttgt aacccaaatc tctcattacc tccagaatag gtacccaact 840

tctcaggcag ccactgcagt cctgactcct tccccctgct ctttgttccc agctaaaagg 900

aaacagatct atggtttcct caaaaatctc agcttagttt tactaagcac ttgcgcctcc 960

tgataccagt gccagagata acctttcaca agtttccact ggctgacaaa aatgggaaca 1020

cctcagtata acccctgtaa cctctggcat ggacttaaga gccctgggct tggaatttct 1080

ccagggcacc agacccagga ttggggtaac agcacttaag aatactagga aaccacaatc 1140

ccaagaacat gggggtagag gctactgagg gaccgaacac tctccacttc cctatgtgag 1200

ttccatacgc ccttctacaa ctgggagaac cagggaaaga ggaatgcatc cctggtgagg 1260

ctagatgagc tcaagcctcc ctgtagccct gcctggccct gaactcaggc tggctgtttt 1320

actttctggt ctcagtgctg tcacctcttg ccaactgtag ggcaatgaaa aaaagatgta 1380

gcctcccact atctcaatgt cctcatcgcc ccatcgctgc tcttcctgtg aacagtcttt 1440

ggaaaagttt ttaaacccta acatagggcg ggcacggtgg ctcacgcctg taatcccagc 1500

actttgggag gccgaggcag gaggatcact tgtcaggagt tccagaccag tctggccaaa 1560

atggtgaaat cccgtctcta ctaaaaatac aaaaaattag ccggacgttg taatcccagc 1620

tgcaggcttg taatcccagc tgctggggag gctgaggcag gagaatcgct tgaacccggg 1680

agtcggaggt tgcagtgacc cgagatcgcg tcattgcact ccagcctggg cgataagagc 1740

gaaactccgt ctcaaagaaa aaaaaactaa cataaatggc gtccctcctt tgttcagaac 1800

tctccgtggc ttctagcatc ctcacaatga cagtacaacc ctaggagtaa ctccgcctca 1860

tattcttcgt tccctgcaga aaacagcttt ccgaattctc ctggctcagt cgcgcctcaa 1920

cctttgcacg cgccggttcc tccgcctgtc acgctctccc acacctcgtc acacgcagtg 1980

tcaaaaaaag ggccccaccc a 2001

<210> 23
<211> 561
<212> DNA
<213> Homo sapiens

<400> 23
tcgcccggct caaccccgac gtccgcgccc cggccgcctg ttggccatgg cgggcctggg 60

cctgggctcc gccgttcccg tgtggctggc cgaggacgac ctcggctgca tcatctgcca 120

ggggctgctg gactggcccg ccacgctgcc ctgcggccac agcttctgcc gccactgcct 180

ggaggccctg tggggcgccc gcgacgcccg ccgctgggcc tgccccactt gccgccaggg 240

cgccgcgcag cagccgcacc tgcggaagaa cacgctactg caggacctgg ccgacaagta 300

ccgccgcgcc gcacgcgaga tacaggcggg ctccgaccct gcccactgcc cctgcccggg 360

ctccagttcc ctctccagcg cggccgcgag gccccggcgc cgcccggaac tgcagcgggt 420

agggaggccg ggcccgcagc tcccctggct cccccgggct gcccgccgcc tgaccctttc 480

ccatgtggct cgaacccctt tcctcagccg ttctactttt acgttccttt tctcagtcta 540

aaagtcgagt tccgctcttc g 561

<210> 24
<211> 2001
<212> DNA
<213> Homo sapiens

<400> 24
gaaagttcct ctgttgctct gggagagggc gggggagagc aggctcgaga gccaggctcc 60

tccgaggctg gtcttgaggc acttctctag tagcttctcc aaaagactga gagtgccggc 120

gtaggtatga cagtgagggt acctcacaga cccttctcca aagtctggcg ggccttgggg 180

tttttcgggg ccaccaggct cggtggaatt tttgaaacgc tttcgaaata catagtttcc 240

tctgtggagt gagtgcctac aacgcgcagg ccggactgat cccccgttgc tgcaggttgg 300

tgccccaagc tgcgggtgct cgggcgccaa ctaaagccag ctctgtccag acgcggaaag 360

aaaaatgggc tgtgaaaaag caaaaggcct cgtctttgaa tgaaagttaa acattaaaat 420

ctgaccctag agttgtctaa agatcgcgga attttgaagc tccggcagag cggactaaaa 480

aacggtgcta tgagagatgg tgagaatact ctaggcatga acgtgtgcgt gtgtgtttgt 540

gtgtgtgtgt gtgtttcatt cttcccgcaa aacaattttt tgtttttttc ctattcccgg 600

tttgttatcg gcctagggcg ggagaaccac gcagcggctt ctgggcccta aggacaaaag 660

agttaaaaca atgaggctca cccgggaaga gacgctgccc tgggcacaat agggtcgcct 720

gcattactcc tccatacaca catctttaaa tgtgtccctg tgtgtgttcg ttagggtgct 780

gtattacaga aaaagaaagg cctaaaaaca cccccagccc tggtcgcgcc tttcgctacc 840

gcctgagtct ggagccgaca gctccacctc ttctgctccc tggaccgccg cgtctccacg 900

ccacggcgcc ctttttacta aaagatcttt tctcatccta tcagcaaatc gttaagaaag 960

gcttagccat tgcgggggct ccaacttaag gattcccccg gcccactaaa aggctaggcc 1020

cggcctgtag cccagctccg cagaaagcca gagggtgctg ggctttcagc ttcttcctcc 1080

tagacacttg ccccacaaat atatttcgtt ttctctaatc caaataccca tctttttctt 1140

ttttaaaaaa tgataacgta atgggaaatg accaaccgaa ctctgttaca taaagttagt 1200

tctgttagat cttccacccc acccccatcc cgcgggagcg agtaaataga attcatgagc 1260

ttagctcccc aggttcacgc tctggaatgg tttctttttg cctcattccc taagttttct 1320

ctcttctgcc tcctgaatgg agctcaggct aaggagaacg gcagaaagag caaactctga 1380

tctgaatctc taattatgac cccatgtatt acccatttga acataaggcc ctagacgggc 1440

tccgtgcgat ctggggcctc ccaagagaaa acttccccgg gacaggacgt ctgccacgcg 1500

cagctaaaca acttctgttt tttccgccgt ggggaaaata aaagaacctt acaaattcta 1560

aggcgtcata acccctgcaa gaacttctaa ctgtatgaag gcccacgcga gattttgaca 1620

atagataaat gagctgagga aatagggtct ggccagcgaa gggaaacaca cagtagccct 1680

gggtgccttt ctggaatgcc cacgcagggg tccgcgtgga caagcacttg cattcaaata 1740

caggaaaagg cttggacggt cgaaataaat ctccttttaa ttttcttttc atcgactaat 1800

aaaaataatt ccccagcact aaactcaaat accgtaacgg gccacaaaaa cacggagaat 1860

tcataaaact ctatctctgc aggtcacccg ctaatcgcat tattattagc ctcgggagca 1920

tggaaattga actgtcactg cctaaagaga aaatgtaagc gacagctgtc cctcctctga 1980

gttggacagc tttgtggctg a 2001

<210> 25
<211> 2001
<212> DNA
<213> Homo sapiens

<400> 25
gggcagaaat gaaatcaact gtggcaaggc cttggctgct ttcacggagg agtttttctg 60

cgccagtgtc tttttccttc cctttaaaat aaaattaaaa atagcaagca cttctcaggc 120

attcatcaga gatagataga tgcacgagga ttgagtgggc attttcataa agaatgaggc 180

cggctgttat agaccggcgg cctagcagat gaaaacttaa ttagcgtgcc tgtcctaaaa 240

cctaggcata aatctccctc tgccttttgg ataacgctat atctttgctt atgagaaatg 300

ggatgtgagc aactcgctgc acatttctct gattctccag gtcttggtcg gctgacacgc 360

attcgatcaa gtttaaagga atgcgcataa atcagcaagc ccctagcgtc tccttgggag 420

aggtccgcaa atccaggagg gcgcctctga acccaccggg tctggggatt agcagtccag 480

ggcaacctcc gtctctgctc ctgaactcgg gaattcacag aggaagcaag acactgcatc 540

ttcaccaagg cctccaaaca catgcagcag agtgcaatct gcacttacat gtattacaaa 600

gtgaaatctg tgtcaactct ccgcacacaa atgttgcatc tgcagctgaa tttcactgcc 660

tagtggtgaa tttttaagaa aagatttcaa ctaggttgtt ttaatttttt tcttcccttt 720

tctgttaatt ttttttaaaa acccacaact tgaataactt gaatgggtgg cttcagctct 780

gcatcagtca caaataggag tgaaatgcat agcgacattt aacaatcatc cacttaaaat 840

aagtaaataa atatgatagt actgagagca gatagaaaaa gtagcgtttt tttttaaagt 900

cccattttta ttttcttaat tcaggaagag ttttcttttt agaaaaaaat actttaatca 960

ggctttcaac aacattatcc atgggtcagt ggctgatact attattccta tttttcagga 1020

ggtggctggt ctctccttga tttttgtttt tgtttttgtt tttgttttaa ggttttagac 1080

tgattgctat ttgggcatta aaggagccat aataaataat ccatgcccac tttaggttat 1140

ctggtagatc cacagaaatt ttaaatagga ggagagttag gtaagatcga cactatcaat 1200

gaccatttta gaactggggg gaaaaaatcc ccacaacaac cctgaaatgt cttctgtcat 1260

tacagtttca aaaactagag agagaaaaaa agaaggctac tactttaccc agggttcctg 1320

tagtggtgat ggctttcgaa aggggcggga tcccggctgg agagctgctg ttggcctcct 1380

tcctaggctc gaggctcaga atatttctta catctaaaga aaaatatccc ctgtcaacag 1440

aagagtccct tttggagctg ttcttaaaca cacagtttga tccagctttg aggggatttt 1500

ccaccacttt aaacattttg ggagaaagtt gttactttgg cttgatggca gctcatttgg 1560

aaatggagta ctgtttggaa caagaggtgg agaggtgggt ctgaagcaac attatcattt 1620

gtttccacaa gtggagtgaa aatcctcagg gcagcaaaat ataattgaat ttctcgagac 1680

ctttcgatat gtatgtttca acaccagcct gtttttgaga cagctttaga gactctttcg 1740

taattctcat ctataaagaa gttgtgagtc ctcaggagag gttggagagg tttccggcag 1800

ccacttttgt aaccaatcaa tattattttc cataaaatga tgaatctggt tcttccattc 1860

actattactt tcctctaacg taaagataaa attagcctgc atctcacaat tctgcatccc 1920

acggctactg attccaccaa cattttaata catatgcgca tagcatagat ttgacaaaaa 1980

cacattatcc tatgtgtata t 2001

<210> 26
<211> 516
<212> DNA
<213> Homo sapiens

<400> 26
gcggcatccg ggatctggcg ccgcttttgc gtcaggcttc tgcctgagct cggttagggc 60

ctcaccgacc tgcttccacc cctcagggag gcctcagtga ttcggccaca gcctcagcct 120

ccgtcgctct gtgacctgcg ggtattggat gattcgtagc taagactcta cgacatccct 180

gaagccggga aatggtgagt gtgccgggca gggcgtccgg aggcgacgtg gcggggaggc 240

cttatcggaa ccagcgggaa atggcggcag cggtacccag tctgcgaacg gagtccccgc 300

tgccgccgct cagccctcgg tcctcagtcc cctccggtga gggacccgcg ctcctgtcgg 360

gggacccgcg ctcctgtcgg ggtccccgca aggctgctct ggcccagcct gcagccctcc 420

ttgtgcagtt ttgcgcccgc agccccgcac cttccccggg ctgtggggtg aggagtagct 480

catctggaag acgcctgcgt cgcgtgcgcg atgccg 516

<210>    27
<211>    329
<212>    DNA
<213>    Homo sapiens

<400>    27
acgaacgcct cagtgtcccc gaccctgggc agcggggact cgagcaggcg cccctcactg      60

atggctttag aacgtgggtg ggggaaggtg tgtgaggacg ggaagacgcc gcactcacct     120

gagttggcgt cctcagagtg gccgctgcca tcagactctg cgggtagagc tgggccggga     180

gcgacgggcg acattggtag ggacccgggg acagcggtcc ctatcccagg cctgacgtgg     240

gtcccccagg gcggcgtcgc caaggcttag acgctttcgt gcaggaggga cgacgactcc     300

cctcacgcct tcgtggcccc aactcggcg                                       329


<210>    28
<211>    4240
<212>    DNA
<213>    Homo sapiens

<400>    28
ccgcagaaat tactcgtgcg caccatttcc gctgtggggg cattcgtaca agtttccgct      60

gcacacacag cctcccgggc cctctcctcc aaggctctgc cggatcttcc aacgaaatcc     120

cagagcagcc tgcgctgggg agcccgcaag tctctccaga tctctgcacc ccgcaccgcc     180

cggaatctgg gacggcgccc acgcagggct gggccaaggg cagagctcgc accctgcctt     240

cacgcccggt tcacttgcgt ccacgaaagc agcgtgccgg cctcctccat cttcccactc     300

gcgcaacgca cggcgacccg cgcgacactt ctgcaatctg aaggcttgct tcttacaaat     360

aaagggccag agtctcacac ttgccttcgt tggagggact tagaagatcc tccccacgtc     420

cacaccttgt aggaaatgca aaacagatcg atgaaattaa acagttgcat ttggaagccc     480

cagaaagacc taaagacatc gtgccggttt gttggagaga gggttgcggg acagggggag     540

cgggccttac gcaacagaaa aggtgggcac agcgcgctca aaatgaccca gtgaggagtt     600

ggtgccgccg ggccagaggc tgcgagtcca gctggctctg gacttgctcc gcaggcgtca     660

gacgccgtgg gaacctgtgt ctgcttcttc tctccaaagt gtatcggtta aaaaaaaata     720

aaagtagtag tagtagtagt ggtaaggaaa aaaataaaaa taaaaaggag acacaattaa     780

ccaggtcata aaagctaggg caccttcgac cagggctctg gccctccagc gatcgttttg     840

cgttgtttct cttctcaaaa gtagtctcag acccctgcct ttccgctgca gctctgcgac     900

ttccccaaac tccttaatcc tgtaaattct gcaagaaact cccatcctgc aagctgcttt     960

tccccctccc ccctgcgttc cttttttctc tccccacccg cgccgcctct ctatgcccct    1020

ctcttctcag aaaaattcct gccccccgcg cgccccaaag cccgggctgc aaacttttcc    1080

ccgccgggcg cctctgcgcc agatgccgga gcgtctccac aaagcctgag catctgcaca    1140

agttcgcagc ctaactgcgg gataaagacg tttcccccgt agcttaacta gaaaagcgcc 1200

atcgatgggt gtgttaaacg ggataactag agatttcaaa caccttttat ttgcctgtct 1260

tgaaaaaaaa atctaaatga atacgcccgc taccaaaagg caaaataaaa ccaaccttaa 1320

gggtttttgt tgtttttttt ttttttcaaa agtggcgata gggactgttt ggacctgact 1380

ccaacctgcg ccctcccttc ctctatgacc ctcctgcgct tttcctggaa cccaaagctc 1440

tgacttcgtc aaacttacac aattaaaggc aggcggaaga acgcgggctg ggaagcaagc 1500

gggaagattc tagaatggaa gggagcccgc cgagcgccgc gagccgcgcc aggccgggtc 1560

cgatggagca ggcggggatt cctcccccag gcggaccccc gccaccagcc ctgccgggag 1620

ctcgcggcct gcggagcgcc cgggctggcc gctcaccgcc cgcttccccc agcgaacgac 1680

tcggggaagc tccaggaggc catctgtgct gacggttcac accagacagg accacttgca 1740

aggacaaaaa taagaaattt aggaaacgaa aaaagacgta ctggggcgag gggcgcgggc 1800

gcggcgacga cggggccggg ggcacatcct ggcggccgct cggggagaga ggacacgcgc 1860

gggaaggagc gcggcgggtg cacggccgcg ggtgggagta cgcgcctgtg cgcgcggggc 1920

gagggcgagg gcgcgtgcgt gtgaccgcgg ggagggggcg ggcgcgtgtg cggggagcgc 1980

gccgcgccag gggccgagtg tgtggggccg atccagaagt gcgcagcccc ctcacctggc 2040

ccccgtgtca tccccgaaat cccgggaaag ggtgggccgc gcgcgggagt ttggtggagt 2100

tggaactttc ggtcgcgctc gctgcccact ccgctggcgc ccggtggccc gtggtgaagg 2160

gggactaggg tggggaacac cggggccctg cggtcccctc cctttcctgt atttaagaag 2220

ccgccggcgg cgcagaggcc caggcgggct ggcgcggggg cgaggcggcc cggtggcagc 2280

agcgggcggg gcgggcgctc cggagtcggt ggggcccgcg ggttgggggg cggggagagg 2340

ggggagtgga agggaggggg aacgcagggg agggagagga ggggaggagc cgcgcggccc 2400

gcgccgcttc cgaaccggaa agttggtctt gccgaagtcc tgccaccccg gcgtgcgcac 2460

tccgctccgc tccggccgcg agcctccgag cccggccggc cgccggggga agcccgcgga 2520

ggggacgcgg ggccgggcga gaaggtccgg agagcggggg gcacctgagc ccgggcgggc 2580

ccgccgcgct gagcggcgct gagagccgcg gcggagcagc gaaggcggcc ggccgacccc 2640

gcgcgcccgg aacaggaggc gcggcgcccg agcggcccgg gcgagacaaa ggcgccgggt 2700

cggagccctg cccgcggccg ctcgctccgg gaggggccgc ccggcggcgg cggcgggggg 2760

ggcgcgggcg gcggcgcaga cactctataa aggggcgagc ccggcgcgcc ggcggagacg 2820

gcgccgcgcg gacgccgcca aagtttgctg cctgcgccct gcggagggac ggccaccgcg 2880

gcccgcgccg cacccgggcc ccgccacagc cgcacccggg gcggccgagg agcgcggcgc 2940

cggagcccgc gatgtgaggc ggcgccgggc agcgcgcgcc ccggtcccga ggcgccgcgg 3000

ccccctcctc gtcggcgcgg ccgctaattg cgagcgcggc ctcatttgca taggccgccg 3060

gagtccgctg gagcccggcc aatcggcgcg gccctccgct aatggccatg cattattcac 3120

cagcctaatt gctcagcccc atgcgcggcc cgcgcagccg ccgccgcccc gcgccccgcg 3180

ccgcgcgccc gccaggccgc cccgcgccgt ccccgccggc cgccccgctg atgccgctgc 3240

cccgcgcggg gcccgagcgc cgctagcagc atgtctcggc gcaagcaggc caagccccag 3300

cacctcaagt cggacgagga gctgctgccg cctgacgggg ctcccgagca cggtgagggc 3360

cggggctgcg gggtggccgg ggggtctggg gctgcccgtc cgggctgggg aagcgcgtgc 3420

ggcgggagcg gatgcgcgcg tccgggagcg ggagaaagtt ccctgcttcc tgcgggcaag 3480

cgtccgcccc gcgccaggcc ggccgcgggg ccccgggtac ttcgccggag cgcgcgcggc 3540

cgccgagaga gttgtgggcg aagtaaactt ggctcctctc ctcggagtcg gggagctgcc 3600

cgcgaagggc gccgaggccg cggccggctc gaggacggct cggaggccgg ggcgggaggg 3660

agtccacggt gcctccgccg ccgcgccgcc ccccagggtc tctgcgccag gacgctgagg 3720

ccggcggcgg cggggaaggc gaccgcagcc cacctaccgc tggacgcggg ttggggaccc 3780

cgccgcccgg ccagctttgt tcgggggccc gcggcccctc ccgggccccc gcaccgcctc 3840

gggtgacccg cggtgtccca gcgcgttgac gcagcctgtg atccctcgcg aggcgaggag 3900

aaggtcgggg gcttggctct gcctaatggc cgcccgggga attaagctgg gggtgagcgc 3960

agcggcggcg gcctgggcct ggcccctgct cgcggcgtgt ttccggggcg ttcgttgcag 4020

cgtctgcgcg ggcctttctt ctcccgtctt tttggatccg ccgaggccgg gcgctggaga 4080

cctcggcttt gcagtcattt cgctggtagg agcgtcctct tcgaaacatc caagagcaaa 4140

gggcaggcgc cgcgaaagtt aagagactgg caaagggctg gacttcccag agtggcgcct 4200

tagccccgca aagtttgggg cgcccccacc cccttcgtcg 4240

<210> 29
<211> 2188
<212> DNA
<213> Homo sapiens

<400> 29
gcgcgcgcgg agcccgctga gacttgaatc aatctggtct aacggtttcc cctaaaccgc 60

taggagccct caatcggcgg gacagcaggg cgcggtgagt caccgccggt gactaagcga 120

ccccacccct ctccctcggg ctttcctctg ccaccgccgt ctcgcaactc ccgccgtccg 180

aagctggact gagcccgtta ggtccctcga cagaacctcc cctcccccca acatctctcc 240

gccaaggcaa gtcgatggac agaggcgcgg gccggagcag cccccctttc caagcgggcg 300

gcgcgcgagg ctgcggcgag gcctgagccc tgcgttcctg cgctgtgcgc gcccccaccc 360

cgcgttccaa tctcaggcgc tctttgtttc tttctccgcg acttcagatc tgagggattc 420

cttactcttt cctcttcccg ctcctttgcc cgcgggtctc cccgcctgac cgcagccccg 480

agaccgccgc gcacctcctc ccacgcccct ttggcgtggt gccaccggac ccctctggtt 540

cagtcccagg cggacccccc cctcaccgcg cgaccccgcc tttttcagca ccccagggtg 600

agcccagctc agactatcat ccggaaagcc cccaaaagtc ccagcccagc gctgaagtaa 660

cgggaccatg cccagtccca ggccccggag caggaaggct cgagggcgcc cccaccccac 720

ccgcccaccc tccccgcttc tcgctaggtc cctattggct ggcgcgctcc gcggctggga 780

tggcagtggg aggggaccct ctttcctaac ggggttataa aaacagcgcc ctcggcgggg 840

tccagtcctc tgccactctc gctccgaggt ccccgcgcca gagacgcagc cgcgctccca 900

ccacccacac ccaccgcgcc ctcgttcgcc tcttctccgg gagccagtcc gcgccaccgc 960

cgccgcccag gccatcgcca ccctccgcag ccatgtccac caggtccgtg tcctcgtcct 1020

cctaccgcag gatgttcggc ggcccgggca ccgcgagccg gccgagctcc agccggagct 1080

acgtgactac gtccacccgc acctacagcc tgggcagcgc gctgcgcccc agcaccagcc 1140

gcagcctcta cgcctcgtcc ccgggcggcg tgtatgccac gcgctcctct gccgtgcgcc 1200

tgcggagcag cgtgcccggg gtgcggctcc tgcaggactc ggtggacttc tcgctggccg 1260

acgccatcaa caccgagttc aagaacaccc gcaccaacga gaaggtggag ctgcaggagc 1320

tgaatgaccg cttcgccaac tacatcgaca aggtgcgctt cctggagcag cagaataaga 1380

tcctgctggc cgagctcgag cagctcaagg gccaaggcaa gtcgcgcctg ggggacctct 1440

acgaggagga gatgcgggag ctgcgccggc aggtggacca gctaaccaac gacaaagccc 1500

gcgtcgaggt ggagcgcgac aacctggccg aggacatcat gcgcctccgg gagaagtaag 1560

gctgcgccca tgcaagtagc tgggcctcgg gagggggctg gagggagagg ggaacgcccc 1620

cccggccccc gcgagagctg ccacgccctt ggggatgtgg ccggggggag gcctgccagg 1680

gagacagcgg agagcggggc tgtggctgtg gtggcgcagc cccgcccaga acccagacct 1740

tgcagttcgc atttcctcct ctgtccccac acattgccca aggacgctcc gtttcaagtt 1800

acagatttct taaaactacc actttgtgtg cagttgaagg cccttgggca caatgagagc 1860

cagtcctcca aactttcaga aagtttcctg ccccttctgg caggctgcca atcaccgggc 1920

gggagaagga aggaggggaa ggcggtggag ggagcgagac aaagggatgg tccctcgggg 1980

gcggggatgg cggggctgtc ctgtaggtct gtgcggccac cgtgattgcc cctctgcgcg 2040

gtgcccgaag tcccgctgaa acctgccgag ggcagcaggt ctgaaagctg caggcgctag 2100

ttgcgcggag gtggcgcagc tgctctggag gcgcagagcg aatacgtggt gtttgggtgt 2160

ggccgccccg cccctggcgg tttcctcg 2188

<210> 30
<211> 2933

<212>    DNA
<213>    Homo sapiens

<400>    30
gcgccggtcc ggagccggag cgcgggaatc actcgctgcc tcagcccaag cgggttcact      60

gggtgcctgc ggcagctgcg caggtggaga gcgcccagcc tgggaggcag tagtacgggt     120

aatagtagga gggctgcagt ggcagaagcg agggtggccg cagcacttcg ccgggcaggt     180

attgtctctg gtcgtcgcgc accagcacct ttacggccac cttcttggcg gcgggcgccg     240

aggccagcag gtcggctgcc atctgccggc gctttgtctt gtagcgacgg ttctggaacc     300

agattttcac ctgcgtctcg gtgagcttca gcgacgcggc caggtctgcg cgctcgggcc     360

cggacaggta gcgctggtgg ttaaagcggc gctccagctc gaagacctgc gcgtgggaga     420

aagcggcccg cgagcgcttc ttgcgtggct tgggcgccgc cggctcctcc tcctcctccg     480

cgacgcctgc cggcccgctg ccgcccccgc cgccggcccc gctgcacagc gcggacacgt     540

gtgcacctct ggggccaaca ccgtcgtcct cggtccttgg gctgcggtcg cctgcggacc     600

ccggtgggaa cagaaacaag agactgtcag cgccacagac gaggtgaggc cgggcctcaa     660

ctgcaggggt cacgggagtg gggcggaaat acactttgat cccactcaag cggagcggag     720

gtctgggagg ccctgggccc gggagaccag tcttagactc ttgccccact gggtatccca     780

tctaggcctc ttctggggag ggcggcagac tcagccgctg tgtcaacgct gtgttgtcga     840

gaccagctcc ccaccctctc tgggccccag gctcccctca gtaacttggg gcactcgacc     900

cgagcatccg cgaaagccct cccggctctc agcgttgagc attgggattc tagactgcat     960

ttccgtctct ctgcttgggt tcacgcgcct ctccacactt agttcacacg cacacacgcg    1020

cgcgtcctcg cagcacacac ttgtctggtg caggtaaggg aaggtggagg cggatcctgg    1080

ggccaaaggt atttagaatc tttcaccctc agccgcctgg gattgctgtg agagacatgg    1140

aaacaggctg agccgaggcc ttagatgaga ggatggactg gagagtaaag agggagggtt    1200

gcccctgcat cgagtttttg gaccctgatc ccacaccagc ttctcggtct cgtacccgcc    1260

cttccgaaga actccagcag aaaggtccag cggtcccctg tgcttgaggc ctacagaagc    1320

ttgtacccaa ctagggcagg cacccgggtc ttccagacca caggacagga caggccacgg    1380

ctgaggaggc ctctctcctg cctccaggat gaactaaaga cccaatccgg gatcttcggc    1440

ctagggctgc tctcccagac ctggggtctg agaaagccaa accagccctt tccccaaagc    1500

tctagttctg cagattctca gctctggccc actcggaggt gttcttcacc acctatccac    1560

ctactgtggg gcccggccct gggaccttga actggcaggt ctctggtcca gagctaggtc    1620

actggctacc tgaggtctct gaacccctca cttttccgct tccctgattt tggggatttg    1680

gggacagaca cggcagaaag cactggcgac gaactcaaaa actcccgaac gcaaggggca    1740

gcggttctcc caacccagtc taatgcacat tggcccagga tgtctcaggc ctcaccccag    1800

gacgtagggc tctgaggagc tactccggtc tctcgcgggc tcagttcccg aagtgataga 1860

gcagctcgcg ccagagcgca gaacttcggg atttggccag cctccgagcc ccagggcgca 1920

gggtgctcaa gccgaccacc ccactcggcg tggttgccct ccgcgtccat cccctcagcc 1980

cggcccccat ccccgcgaag ccgcagcaga cctgagacgc tggcggacat ctcgctgtcg 2040

ctccggcccg cggcttcctc ctctaggtct ttggaagcgg ccagctcaca gaccggctgg 2100

ccgaggctca aggatccccc cgcaaggccg gccccgctgg ccccccgcgc gtccgcgcag 2160

cgccgcctgc tctcgttctc ctcgctgagc gcggagtccg agtcccagcc ttccgggctc 2220

tccgcagtcc gccccgcagc tgttctggta ccggcaggag acgccagcag agagtcctcg 2280

gcgcccccca acgcgcccgc gtccctctcc ccaaagagcc gccaacagca gacagcggga 2340

gccgcggcca ccgatgccgc tgtgcccccg ggcgccgggc gcccctctgg cgcggccagc 2400

ccgccgcgct cctctttctt gttgaggatc gcctggatgg agaaggacgt caaggtgttg 2460

gcgccgcgca cagccatctg cgccgcgggc aggagcggcc ggcggggcgg gcagctgggg 2520

cgccgagcag ctccgagcgg gacagagagc gccggcggcc gcagcgcgag tgagctgggt 2580

gtgcgaggcc gccgccgccc actgctgcgc ggcccagcag ctcccgcccc actccgtccc 2640

aggatcagcg ccgaccctcg cccccacctt agaggcccac cccgcccgga gaccccctcc 2700

ccccgaatcc agagccagac gctctccttt cgcagctcag ctggattatc tcatcgcttc 2760

tcgcccttag gggcgggctg gggtctgccc cctcggggga cgtgaaggag gattggcggg 2820

ggcccctccg tggcagcagt cccctcccga gcgccgccgg ggcgcacagc ccgagtcact 2880

ttttctttgc gcgtctgtcc cttcctcgcc tgcaggattt cgctcctggc ccg 2933

<210> 31
<211> 2001
<212> DNA
<213> Homo sapiens

<400> 31
agtgtcacat caacaaattt acacatcaat ctaccgcagc taacttcgta acaatgggag 60

aaacattcag aataatactg agcatcctac caagggtctg aaaaattgaa ttcaaatact 120

ctgtgtgtaa aatgcctaga ctctgtcatt ccagcacatc tatgatctga tctagcaagt 180

atatcgttag actacaaatt acctttttcc tatgacgtgt aaaactccat taaaaatgaa 240

ttcttcctaa taaagttttt tatggcgtct aaaattgctg tgaatgttac accttttaca 300

atcacctttt agccagaaag ccattatttg tagaatcctc ctgtatttca gttatttgtc 360

acctatttag gctgggccta atagcaaaac tgtcccccgt tactgaattc agagaattat 420

tcgggcacac gatttatttc ctatcttgat tagactcctg agcccgtgcc ccagcctctc 480

gctaatctcc ctggaccaga caactccatt agaatctggc acccacgttt gttctgccta 540

acactgcagg aaggacagag acttcaaagc acgtgtttgt ttttttgttt tgtttttggc 600

taccaagaag ccaaatttct gtatcctcta ccattcaaaa ccccaattca acaaatttac 660

acgggggttt ttcctccacg ttaagcagtt agtcgggtac tagagataca catataaaac 720

acagactctg ccctcaaaca acccaatgag cagaaaattc tcttaggcac caaaacgctg 780

taatagattc aagtgtgtag aggagaagtt tggtagagtg gatatgacgc tttctttctt 840

tgtagtacag aaaagataaa tctgtagaaa agggagaaag acaactgggt agaaaattta 900

tttcaaatat ctaacccaaa tcttcaacag attttccatt ttaaatattc caaaaagtgt 960

accattgtat attatactaa atgcaggttc atttatcact taaaaatttt taagctaaaa 1020

aatctcaaac aattaacatt tgggaagaaa aacaggactg atacacaaag tagtcaaaat 1080

atttcagctt tctaaactgt atgcactgga ctaactgttc aatattagaa tatctctaca 1140

tttgaatttg gatagcccac agtgataaat actggactga aaaatctgac atcgaacata 1200

tgcaaaacta atggctacta tgaaaaaaga tagaatgggg agagaaaact tgaatgtgcc 1260

aaaacattta aacgctcttt aaaatatcct gagatgctaa attaaggaca aaacgattag 1320

agttccaaga atacaaattt tcatctcttt caagattcaa ctgaatattg aatctcattg 1380

agattatgaa atattctcta agcatgtgct taacttctat ttggctttcc gcatttcacc 1440

acagtgaaca gcccattctt tttccttgtt tacaccaaat gctcgttttg aacacaactc 1500

aaaatggaat tccaggccca aaagtcacca cccctacttt cacccccaca ggcagctact 1560

taacagataa ggaattcaag tgcaggacct gaaggtctta tttccatgca aatttcacaa 1620

tccccgttac ttgcccagat acaacaatta aagcttaaaa ggtggcggga gtgggggact 1680

tgaggactgg tctgaggaga aagtgaatct cccaagggtt cctaaatggt tttgcttcca 1740

gtataaaaac tgcgagctac cagtagaatt taacaacagc tcaaccttgc atttggaaca 1800

gttactatat agttcacttt cttttttcat gggggcgggg tatggtgtct tacctactct 1860

taaatttgaa cgtattaaca ggttcccctc cgcgcacact gacatatttc ttatccccca 1920

taatgaattc agccatatgg cattctttcc catcgaaggc catcgggaat ggctttagga 1980

agctgatttt caagctttaa g 2001

<210> 32
<211> 567
<212> DNA
<213> Homo sapiens

<400> 32
tcggcggccc ccccgctgtc tggcggacac ttgttagtgg ctgcggagaa gccactcaca 60

aagtttccca tcccgttgag ggaaggggtc ctgactgcgc cagcggggca ggcccagaag 120

gcgcggtatc tgggaggtcc ggccgccgca gacgaccccg ccgaggccca aagtgcgcca 180

gcttctccgc gcccctccgc ctcctcctcc tcctcctccc tgcagagggg cgcacgcgca 240

cagacacacg cacgcacgca cgcacttaca cacaaaagga agtcatggaa ggtgctggtc 300

cctgcataca ggcacactcg cgcgggacac acacacaccc cccaaaaaga ggcgtgcggg 360

gttcgccaga cggtgggcaa aagcccgtcc tccccccctt ccagggcctg ctcacttcag 420

ggagcgccca ctcgcccagc cacgggccaa gagcgcacgg acccaggcgg gcggcagccc 480

acccgccacc acgcagctcc acttcgctgt tccacagcca ccaaccgcac agccggcaca 540

gtcccgcccg cgcagctggc ccaatcg 567

<210> 33
<211> 1001
<212> DNA
<213> Homo sapiens

<400> 33
ggcatagttt aaaactatcc ctgctcattc tttaaaataa gtccacagta gagaataaga 60

catcggaaaa tacaaacatt tcttcatatc cgaatctatt tgaatcctaa gatgcagata 120

cggagagttc agagtgccat cagtacaggg cagagaggtt gaagagctca ggaacagaca 180

tagggtgggg gaaaggggta ggggcaacga cgctgacttt tggttaacaa agcccttcca 240

ggctgcggag caacctcctc tgcccttcac ctgcccggcc catctctggc caagaagacc 300

ctgccgccaa atccccacac ccagtccagg tcgcagtgca cagactggcc cttccgaagc 360

ccctcagcgg tagcccgact ccgaagctca ccgaggcatc cgtgagagga gatgccacct 420

agcgcagatc acatctgctc tgaatccttg acaaccgcag cccaaagaat gataaactac 480

aaaggccgga aatgcgtcac cgcggcccgc tctccgcgaa acagcggttc cggctgtgtt 540

ccttctagga aggccggagg tttccacacc tctgtggtcg tcactctgaa tcccgtctgt 600

agtcttaagt gagatactag gtgacacatt gtcttccacg cggcaatata ataacggcca 660

acatagtgtt ttaacacgta ttaattcatt accccgcata acaaccctgt gagttaggta 720

caattatctc catttaacag gtgaggaaac tgaagcacat ttctacattt attagttgcc 780

atttcctgca aagaataccc tttcttttcc ctgccgtctc attttatcac gatgaactca 840

tggattcctt tacaaataat tactgttatt attatgttga tgctcaaatt atttaaaatt 900

tggtcagttg gagccctttc acactgctcc ctctcttttc tttttttgac aaagtctcca 960

ggctggagtg cagtggatgc gatctcagct cactgcaacc t 1001

<210> 34
<211> 383
<212> DNA
<213> Homo sapiens

<400> 34

tcgcacgttc gcaggcgcgg gcttcctgtg cgcggccgag cccgggccca gcgccgcctg 60

cagcctcggg aagggagcgg atagcggagc cccgagccgc ccgcagagca agcgcgggga 120

accaaggaga cgctcctggc actgcaggta cgccgacttc agtctcgcgc tcccgcccgc 180

ctttcctctc ttgaacgtgg cagggacgcc gggggacttc ggtgcgaggg tcaccgccgg 240

gttaactggc gaggcaaggc gggggcagcg cgcacgtggc cgtggagccc ggcctggtcc 300

cgcgcgcgcc tgcgggtgcc ccctggggac tcagtggtgt cgcctcgccc gggaccagag 360

attgcgctgg atggattccc gcg 383

<210> 35
<211> 279
<212> DNA
<213> Homo sapiens

<400> 35
ccgccagggc acggcccccc ctgcgcccca aactgagcgg caaagtcagg gcccgcggcc 60

ggatgctcag agctaaaggc cgcggaggac agatgtgctt cttcctcctt cccgcgtctc 120

cccatacaag tactaccccg cacgtcccat caggcttgcc tgtgggccag gattcagggt 180

cctgagccga aacctaccag gagagagaag gctctggaga cctctgtaac agtcgtgcgg 240

agaagacaaa gtcagctgcg tgcgtctcct ccggcgccg 279

<210> 36
<211> 2775
<212> DNA
<213> Homo sapiens

<400> 36
tcggagtcac gtgagcgccg aggcccctcc cgcggcaggc ggcgaaaggg cttgcgcgcc 60

ctcccctcct ccacagcccc ccgcccctcg cgggcccgcc cctccaggcg aggccaacct 120

ccgcgcccgc cgcccgagcc tcagcggtcc gggaggagct cccggcggcg ctcggcagag 180

ccctcggccg gtgccccgcg gccgccgcgc tcccagggct actggcgcag cgcacggaga 240

acccggttct cggcgcggtg cgtcgtgctg ggcccccgcg ccgggccacc tgaagccaga 300

ggatttgggg cgcactgaag ggactgcgtc tcccagctcg aacccggctt aagtggggcc 360

gggagcgagg tcgggaaagt ctcacccgcc caaagcctca ccaccgagag gcacttaaaa 420

aggaaagcgc agagggaccc tgcccacgcg cgtgtacaca cacacccccc cacacacaca 480

caagcaaaca cgagctcccc gccacttcct ccccagggtc tcctcaaggc caaatattgc 540

tcccaatgac agccagtcac cccttggcga acgcctgcta aggctccgaa gagccgggcc 600

accgatctag ctcccggctg aaagcagccg accttgtcac gcgcggggcc gggaatggga 660

gggagggtgt tagagggtga tcgctgtggg aaagtgagag ggagcggctg ttagtcattg 720

ctccgggtcc attaccgaga atccccaaac ctagtccgcc gctgcgtggc ccctctcccc 780

atgcaaagca gacccccgaa gaagccatgc caggctgagg gacagacgcc ggggctcgaa 840

gctccgggca gattcagaaa gaggcgtcgc tgcagaaagg acgcatcaca gttttcagat 900

cttaatgtgg ccgaggtttt acaactcccg acccggcgca gaaaggaaat cccaccatgt 960

tccccggagt cgagaaaacg gtgaacagct ttcggcctgc gctcgacctc tgcgtctgcg 1020

tctctctcgc ctcggcttcc cttatttttt aaaccaccac cacactcctt cccccgccac 1080

ttccttcccc cacccccttc ctccgttgca ccagcagcag agtcgcacgc agcaaatact 1140

ccttcaagaa ttttacctac ctacagttca agcagttact gggatgtcct gactaatcga 1200

agatgctgcc gcgcgcgtgg gtcgctctgc gcaagggcct cttcgaaaac ccgactaggc 1260

gcaactcagc gttcagcagg gccgggagcg ccaggtcgtc cccggggccc gggccccatg 1320

actcctgccc caaagcccac tccacccgac ctccctttcc tgaggctgtt cccagttgct 1380

gctttgggtc gctccggagc tcaagaactc gggttgcctg ccgccccact ctccacgcac 1440

atacttggtt ttcttcttag gggcattggc aggtagactt tgaggaagaa aagtaaagga 1500

tcgaacagct cagccctccc tcccgaccgt ggatgcccgg agtcgaccaa cacctcaggt 1560

ccgggtgcgg aggccgcggg cgcccctgcg cgaccgtccg cgcccggcaa gagccgcgcg 1620

gctttcgcct ttgctggtcc cgcgccaccg ctggggcggg ctgcgaaagg gttgggaaga 1680

gcaaagggtt tttttgtttt gttttgagac gcagaagccc tttaaaaagc ccggcgagga 1740

gaggtccaga agtagagaaa gcagacggag gcaagctgtg cccgcggggc aaagggacag 1800

tagaaggggc gggcgcccgg gttccccgga aaaccctcgg ccccaaggaa tctcctgggg 1860

cgggagagcg cggttctaaa accgagagga taggaagggg aagggggagt tgtgtttcaa 1920

tttcggattc accaggattc atctctagtc acatttttct tctcaaattt ttaaatcgaa 1980

aagataaaag ccaaaagaac tttcatcccc agagcttttt attgggggaa aggaatgtaa 2040

ctcggggtgg ttgtccttca cttccctact cgaatcttct cctaatgccg aaatgtgttt 2100

acaggtagcc tcagtttacc aagtatgtat cttttggggg tttaacctct cacaaagcct 2160

tcaactcaca aaccgcgatc cttggaaacc atcctccaaa gcagtgcttg gaggcctcta 2220

aggcccccgg accaactccc gctggaagaa gcctgcaggg actcgggaat cacgggaacc 2280

tttcccgtcg gttccgggcc tggagggcca ggaagagccg cgcgtccgcc tttcgtcccg 2340

ccaggaactc cccataggac acgacaccgc aggaacaagc gtcctgggag cccctgggat 2400

cttggctgtc gtctctaggg accctacacc gtgaaatgat agaggcgagg ttccttgggt 2460

tccgcaagtc gacgaaaata gctcgtggag aaggcgcgtc ctgcaactgc agttcgcaag 2520

ctctcagggc gccccgccag ctgggggcca gattgggtga cactcccctc gacgcagcct 2580

ccggagcggc gcgcactctc cagaggccag caggactgcg ctctctaccg cagaacctgc 2640

tccagctagg tgttctctcc ccatctcgcc gtcgctctgc cccctcactc tctctggacc 2700

tcagagccgg ttctctcctt cctcctcccg cgctttccgt ccggggatcg caacctccag 2760

cccgtgggca acgcg 2775

<210> 37
<211> 2255
<212> DNA
<213> Homo sapiens

<400> 37
ccgctttaga ggcagcgctt atagcgctag ctggtcgtgg aatgcgatta cagcgtctcc 60

attggagacc gctgagtgcc tcggtttccc tgtctgtgca aagtgcactc cccagacgcc 120

gctgcctcga gggaccagga aatgcgtctg ggggcgccag gaaagatgag aagataaagt 180

cacgatgcgt ccagctagct atagacacaa gcagaggagc cagtaggcca aaggagacgc 240

acagctgatc cgtgccgagg cgcgggctcc actccctgaa gtggagggac ccttgaatct 300

ttccttgcgt aggcgcgcgg cagagcagcg atttggcgaa aagggccgag actcaggatg 360

cctgcaatgc gagcgagggg cggacagggc gcacggggcg cggcaaggct gcgaggggcg 420

ggcctgggcc ctgagcctcc tgcacttcca gccacagctc tgggccttgg gggcgggaag 480

gggtggagcc acgtggggag gagcaaaacc cggaggtccc gggcaccttg ggcagagcca 540

gagcggcggg agccggtcct gggcgcgttg ccccgggagc gcccgtcgtc cgggcagagc 600

gcagccgcaa ccgcgaccac agccgcagtc gctttccagc ctgccttcgg tgcgcagcgg 660

gggaacaggg ctagtgcagc cgccggaggg gggcacgggc tcctctccca tcccagagct 720

actgggctgc ccttgctgtc ctcgccgccc cagcagaccc cggccggacc tgccacctgc 780

gccctggttg cgccatggat ccttcggaaa agaagatatc ggtgtggatc tgccaggaag 840

agaagctggt gtccggcctc tcccgccgca ccacttgctc cgacgttgtg cgagtgcttt 900

tggaggacgg ctgccggcgg cgacggagac agcggcggag ccggcggctg gggtcggccg 960

gcgacccgca tggcccggga gagctgcccg aacccccgaa cgaggacgac gaggacgacg 1020

acgaggcgct gccgcagggc atgctgtgcg ggcccccgca gtgctattgc atcgtggaga 1080

agtggcgcgg ctttgagcgc atcctcccca acaagacgcg catcttgcgc ctctgggctg 1140

cctggggcga agagcaagag aatgtgcgct tcgtgctagt gcgcagcgag gcatcgctgc 1200

ctaacgccgg cccccgcagc gccgaggcgc gcgtagtgct gagccgagag cgcccctgtc 1260

cggcccgcgg ggccccggcg cggcccagcc tggccatgac ccaggagaaa cagcggcgag 1320

tggtgcgcaa ggcctttcgc aaactggcca agctcaaccg gcggcgccag cagcagacac 1380

cgtcgtcctg ttcgtccact tcgtcgtcca ctgcctcgtc ctgctcttcg tcgccgcgga 1440

cccacgagag cgcgtcggtg gagcgcatgg agacgctggt gcatctggtg ctttcccagg 1500

accacacaat tcgccagcag gtgcagcggc tccacgagct ggaccgcgag atcgatcact 1560

acgaggccaa ggtgcacctg gaccgcatgc ggcgtcacgg ggtcaactac gtgcaggaca 1620

cttacttggt tggggcaggc atcgagctcg acgggtccag accgggagag gagccagaag 1680

aggtggcggc ggaggcggag gaggcggcgg cggcgccccc tctagccggc gaggcgcagg 1740

cggcggcgct ggaggagctg gcccggcgct gcgacgactt gctgcggctt caggagcaac 1800

gggttcagca ggaggagttg ctggagcgcc tttcagccga gattcaggag gaactcaacc 1860

agaggtggat gcgacggcgc caggaggagc tggcggcgcg ggaggagccc ctggagcccg 1920

acggtggccc cgacggcgag ctgctgctgg agcaggaacg ggtcaggacg cagctcagta 1980

ccagccttta cattgggctg cggctcaaca cggacctaga ggccgtcaag tcggacttgg 2040

attacagcca gcagcaatgg gacagcaaga agcgcgagct acagggcctt ctgcaaactt 2100

tgcacacttt ggagctgacg gtggcaccgg atggggctcc tggctctggc agtccctcgc 2160

gggaacctgg gcctcaagcc tgcgccgaca tgtgggtgga ccaggcccgt ggactggcca 2220

agagcggtcc tggcaacgac gaagactcgg atacg 2255

<210> 38
<211> 875
<212> DNA
<213> Homo sapiens

<400> 38
ccgcgccacc cctcggctct ctctctctct ctccctaccc cgcaggatct acaccggctg 60

tgacatggac cgcctgaccc cctcgcccaa cgactcgccg cgctcgcaga tcgtgcccgg 120

ggcccgctac gccatggccg gctctttcct gcaggaccag ttcgtgagca actacgccaa 180

ggcccgcttc cacccgggcg cgggcgcggg ccccgggccg ggtacggacc gcagcgtgcc 240

gcacaccaac gggctgctgt cgccgcagca ggccgaggac ccgggcgcgc cctcgccgca 300

acgctggttt gtgacgccgg ccaacaaccg gctggacttc gcggcctcgg cctatgacac 360

ggccacggac ttcgcgggca acgcggccac gctgctctct tacgcggcgg cgggcgtgaa 420

ggcgctgccg ctgcaggctg caggctgcac tggccgcccg ctcggctact acgccgaccc 480

gtcgggctgg ggcgcccgca gtcccccgca gtactgcggc accaagtcgg gctcggtgct 540

gccctgctgg cccaacagcg ccgcggccgc cgcgcgcatg gccggcgcca atccctacct 600

gggcgaggag gccgagggcc tggccgccga gcgctcgccg ctgccgcccg gcgccgccga 660

ggacgccaag cccaaggacc tgtccgattc cagctggatc gagacgccct cctcgatcaa 720

gtccatcgac tccagcgact cggggattta cgagcaggcc aagcggaggc ggatctcgcc 780

ggccgacacg cccgtgtccg agagttcgtc cccgctcaag agcgaggtgc tggcccagcg 840

ggactgcgag aagaactgcg ccaaggacat tagcg 875

<210>    39
<211>    975
<212>    DNA
<213>    Homo sapiens

<400>    39
   ccgccgggtc actggagtct cagccttccg gaatccgagc cggcccgccc cactccccgc    60
   ccttcgcggt cccgcccacg acctctcccc acgcctcccg ctccggcccc caacctcccg    120
   gtcggacgtt cgttcccggc tctagccggc ctccgcgcct ctggcctctt tccttccggc    180
   cgtcccgacg gagatatttc ttcaatactc cataaataca ccccgccgcg gaacccaccc    240
   ggagtgagac gcccaacacg tcgtcgaact ggggttggcc gggggccgct ccccgccgcg    300
   ggcccgcaga ctcgtggcgt cgccccgcag ctccgcctgg ccgacgggga accggccgag    360
   acccggacac gcacgcccgg gaggacaaaa gcgcgggcgg accccgcagg ctgggacccc    420
   ggcggctggc ccgctccccg agaagggccg tggtcggggg gctctcactc acgagccgct    480
   ggctctgggt cagccctgcc cccagggcag cgctccatca tgaggctggc ggggcgctga    540
   gccgtggcgt cctcgctcct gcgctgcccc tctgcatcct ggccccttcc ctgcacacgc    600
   agagctgcca cactgagcgc ccctcagctt acttaagctc ggcaaggctg gagaaggccg    660
   tctgggtgac cggcggaggg gggatgctgg ggaaggaaga attcaggcag ctgcaaagag    720
   cgcgcgaata tattcattcg acatacctca tgggcgccta ccctgggcct ggtccggggc    780
   gggtgtttgc ggggtggggc cgaagcaggg gcgtcgccga gttgaagacg tgtactccga    840
   gcgctcctgc gttcattcat tcgctgggtg gagagaggaa ggacaagagc cccgcgccga    900
   tcggagggga gcagaatagt aggcacagtt agagggtctt cacggtgcgt ttcggaacct    960
   tggctgcccg gctcg                                                  975


<210>    40
<211>    1284
<212>    DNA
<213>    Homo sapiens

<400>    40
tcgacaaacg caaagcgacc caaaccctgg agggtcacat cccggctgct acaaacctcg    60

gcggggcggc cccgctcttg cggccgggac agcgcagcgg cagcaggggc cgcaggggac    120

ccgcagattg gcacgccgct ccccatcccc gcagcgcgtc tgcaccggag actctgcggg    180

gattgtagcc ggagggcggg ccgggctccg aggcgctgct caggcattgg ggtttgtcct    240

catgagctcc acgtcggcgt gcaccatctc cctcaccagc tcctgcaaca caggggtggg    300

cgtgagggag gagcttctgc cactctctcc tggtgacacc ccaccccggg tgtcggcccc    360

agagaggcct ccgcgtccct cgttccagct cccctcactt ctcccgcacc ccgccttccg    420

ggctttgggc atcgcaggcg cctcaggcgc ccgaccctga gagctgccgc cctgcagccc    480

ggggccccgc agcgggcggc gtgcgcccta agagatactc acatcgaaag cgacccgggg 540

cttccagttc agcttctgtt tcgctttggt gcagtcgccc tgcagaaagt cctagggaag 600

aagaggggga gacgaagcag gcgtgggtcg tgggggtggg ggcagcaggt cccgagcccc 660

gggaactccc accgttccgc tccctctggg cgcacaaggc tccgggtttc cctgctttcg 720

gtccctgctg tgcgcgttca gttgcggctc tcggcgccgt aaatcactag gtcgcggtta 780

agaatgtgct gtgcggaccc gtgaggaccg tgaccgcgat ccacccccag ctacctccac 840

acctcttctc cccaaggcgt cccttgggct cttaatgctt tttttttttt tttttttttt 900

tttttataac atgaagttgt cagggacgct cctatgagaa ctgtttggaa ttgctgcact 960

tctctggcta ggagggaagt gagtaaatca ccaggcgccc ctcccagctg cccgtgtccc 1020

tgcgccgctc agctcctgcc gcagggctgg ccgcgccaag cgcgcgtcct acccaaagcc 1080

accagccccg cggggaaggg actcgggctg tggggcgcga ggccccagga ctcggggacc 1140

cctctacctc ggcggcagcg tgcgaccctc tttctaacgc ggccgtggat gtttcttccc 1200

gggccgcagc caagcgcggt tcttcctggg cggtggcttt gggcttttcg tacccacagt 1260

caagtcagtt cacgtcgcct cccg 1284

<210> 41
<211> 1001
<212> DNA
<213> Homo sapiens

<400> 41
actgctctaa atacttcata tatattaact cctctattct gtacttctgt tcccgtttta 60

tacagcagga aattgaaaca ctgagaggtt aagtaactaa agttacagag ctagagtgac 120

aggagtaaag cttcaactca ggcaacccag acttccagag ttctgatctc cactactaag 180

ctgctagcat agcttttctg gtaactattt ttaattcaaa tataattcga gtgatctatc 240

taacaagtca tcactctgac aactcagtga cttgtaatgt aaaattattc attgtaattc 300

atttaatatt attgtttctc tgtgctgcaa aaatcatagc aatcgagatg taatttatta 360

ctctccctcc cacctccggc atcttgtgct aatccttctg ccctgcggac ctcccccgac 420

tctttactat gcgtgtcaac tgccatcaac ttccttgctt gctggggact ggggccgcga 480

gggcataccc ccgaggggta cggggctagg gctaggcagg ctgtgcggtt gggcggggcc 540

ctgtgcccca ctgcggagtg cgggtcggga agcggagaga gaagcagctg tgtaatccgc 600

tggatgcgga ccagggcgct ccccattccc gtcgggagcc cgccgattgg ctgggtgtgg 660

gcgcacgtga ccgacatgtg gctgtattgg tgcagcccgc cagggtgtca ctggagacag 720

aatggaggtg ctgccggact cggaaatggg gtaggtgctg gagccaccat ggccaggctt 780

gctgcggggg gaggggggaa ggtggttttc cctcgcactg tcttaaaccg atggcctttc 840

cttggcacag ggtccactgc agcatgccaa acgaggaggc aggggcgtcg tccccccgcc 900

ccccactgca gcactggaga tggatttcct gtacttcgga tccagggttt ttgacagaag 960

aggaagaagg gggaggggta gaagtgttaa ggggagtctg c 1001

<210> 42
<211> 859
<212> DNA
<213> Homo sapiens

<400> 42
acgcagaggc cgtggcatct ggccgcagct gggctgcagt gcgtgcgcgc ctggcctggt 60

ggtccgatgg gaagcccggg gcggggcagc cgcggggcgg gggcggggcg tcgcggagat 120

aggccacgcc cctgcccgcc cgcgcaggcg cgctgcgggt cgttagctgt cagagccaag 180

cggcgggctg gcggcgggct ccgacgtctg cgccaggacc tggctggctg agcccggcgc 240

agcagcagca gccagggcag cgcggcccct actccctgtc aggtcgtaga ggcgagcagg 300

gaccagctgg tcgccggccc ctcgggcaag atggggaacc gggagatgga ggagctgatc 360

ccgctggtga accgtctgca ggacgcgttt tcggcgctgg gacagagctg cctgctggag 420

ctgccgcaga tcgccgtggt gggcggccag agcgccggca agagctcggt gctcgagaac 480

ttcgtgggca ggtaagcgcg cagggcgcgg agtaaggatg cggcagtggg gcgaccccgc 540

tgcgggccgt tggaacgtgg acgggcagcg ggagccagag ggtggatgga ccaggcgctg 600

cggtggaatg gggggcagag tggaatgggg ggcagagtgg cggtgtccgt ggggcgggcg 660

gggtcctcca gctctgggca tcctccgtcc cctgccaccc cccgcctggt ggccctcctg 720

cctgcctttc atcgtgcgat acaaagccat ttcctccctg tcctccagtc ggggagtcgg 780

gggaggggtc cgcccccggc tcgaccccca ccccctcggt gcgcgccagc cccgggcagc 840

ctccctgcgt agcgcgccg 859

<210> 43
<211> 2001
<212> DNA
<213> Homo sapiens

<400> 43
   gacagaaaac agccagagcg caccactcac ctgagtgcca ggtaaacacc tgggcgcgac 60
   agggacagga aacaagggta gggtgcggag gctggggagg aagaggttgg aaagggggga 120
   aataaatggg cggggcctag caggtcctgt gcggggctta gggccggggc ggggcccagg 180
   aagactcagc agcgggtggg tgagggtcta aaggcggcaa ttccgggccg ggtgcggtgg 240
   ctcacgcctg taatcccagc actttggtag gccgaggcgg gcggatcacc tgagatcaag 300
   agctcgagac cagcctgggc aacgtggtga aaccccgtct ctactaaaaa tacaaaaatt 360
   agctgggcgt ggtggcgggc gcctgtagtc ccagctactt gggaggctga ggcaggagaa 420
   tcgcttgaac ccgggacgtg gaggttgcag tgagctgaga tcgcgccact gtactccagc 480
   ctgggtcaca acagggaaac tccgtctcaa aaaagaaaaa aaaaaaaggc aattccgagc 540
   ccagacaaac cttaaggagg ggatcctgga tcttcagtta agtgggcgac acctggagtg 600
   aggggcgggg catatgcaga gtaggtgcgg cctacaagcc aaaaaggaga aagagttgga 660
   atggtgggcc tggcttatgc gggtgggcgg ggagagggtg gatcctagag gaggtgaggc 720
   ctaacattgg gcgaagaagg cgggagcctg ggccaatgag ctgacggtag gccggggagg 780
   gggcggtggg gtggggtggg caatgggcaa tgagacggag ggcggggccg ggacctaata 840
   tggcgggtca ggagggtctg gaagacgaag aagagggaca ggcaatgcca ggtctaggac 900
   taggagggag gcgcgggcgg tattagcggc tggaggaggc ttcgggaggc ccggccgacg 960
   gccgccgcct ggtgctaccc acccaggggc gcgcgaccct cccttcggtc tggctccaaa 1020
   gacctagcag cactgacttc acccagctgt ggttccaacg gcgggtccag cggcctcggc 1080
   ccggcgccgt cctcctgctg gcccaacagg cccgccagcc cgcccctgta cgtctgtgat 1140
   tggacggcgg cggccactga tgttcaagcg acaggtcctg gcccgggagc caatctgcag 1200
   gtgttgaggc ccaggctccg agagcgggcc gaggaggcgt ggataccctg attcctaggg 1260
   ggcaggcctg gttcccccga ggaggacccg gcctatgaat gactggagtt ctggggttct 1320
   ggccgaaaga ggaagtggga caggccgggg tgtgatgggg cctagagtca cagagccttg 1380
   cggccctgct gtccctgcaa gaagccagct tctggccagg cgcggtggct cacgcctgta 1440
   atcccagcac tttgggaggc cgaggcgggc ggatcacgag gtcaggagat cgagaccatc 1500
   ctaacatggt gaaaccctgt ctctactaaa aatacaaaaa attagccagg cgtggtggcg 1560
   ggcgcctgta gtcccagcta ctagggaggc tgaggcagga gaacggcgtg aacccaggag 1620
   gcggaggttg cagtgagctg agattgcgcc actgcactcc agccagggcg acagagcgag 1680
   actccgtctc aaaaaaaaaa aaaaaagcag ccagcttctt cctcctattt tgcaaccttc 1740
   tcccgatatc cttgaacatt ttagggacag ccatcactta accatagagc aaccctatta 1800
   agtctaagta gcataatcac attcctgtag tatagatcat gaacctgaaa ttcgaggatg 1860
   aagtcatttg cctgaagaca tacatcttgt aaaatagcca tccgcaaaga tgtagggaaa 1920
   aaggcagcga tctgtggcta cacctcccct tcctcccgga agcagccact ggaacgtttt 1980
   tagctttttc tttttttttc a 2001

<210> 44
<211> 1007
<212> DNA
<213> Homo sapiens

<400> 44

acgcggtgac cttgaccccg gcccaggccc tgctaatgaa gaggaaagcc cgtacgcact 60

cggcctgacc cacggcgacc ctctgtgacc aatcatacta ccaacctctt aaacagagct 120

ccaccgacgc aatgcccagg cataaaaagg ccaggccgga gagaccgcca ccagtcacgg 180

accctggacc cagcgcaccc gcaccatggc cggccccagc ctcgcttgct gtctgctcgg 240

cctcctggcg ctgacctccg cctgctacat ccagaactgc cccctgggag gcaagagggc 300

cgcgccggac ctcgacgtgc gcaaggtgag tccccagccc tggtcccgcg gcgctccggg 360

gagggaggga cccgcagcca caggggcgcg ccccgctccg gcctcgcctg agaactccag 420

gagctgagcg gattttgacg ccccgccctt gaccgcggtc gaggccccca cggcgcccca 480

gcgcgtctca gccccgctgt cccgcccgaa ctccgaaccc cggaccccag catccttgcc 540

cggcgcaccc cggccggcct cgcagggtcc tccgagcgag tccccagcgc cgccccggct 600

cccgctcacc ccgcccgtcc ccgcagtgcc tcccctgcgg ccccgggggc aaaggccgct 660

gcttcgggcc caatatctgc tgcgcggaag agctgggctg cttcgtgggc accgccgaag 720

cgctgcgctg ccaggaggag aactacctgc cgtcgccctg ccagtccggc cagaaggcgt 780

gcgggagcgg gggccgctgc gcggtcttgg gcctctgctg cagcccgggt gagcggggca 840

aggcgctccg ggggccaggg gaggcgggcg gggtgcggc cgggattccc ctgactccac 900

ctcttcctcc agacggctgc cacgccgacc ctgcctgcga cgcggaagcc accttctccc 960

agcgctgaaa cttgatggct ccgaacaccc tcgaagcgcg ccactcg 1007

## Description

### [Technical Field]

The present invention relates to a method of assessing liver cancer-related risk by measuring the extent of methylation at a CpG site of a specific gene.

### [Background Art]

Cancer is a disease in which cell division continues because the cell cycle is not regulated, and grows rapidly as it infiltrates into surrounding tissues and spreads to various regions of the body, thereby threatening life.

Cancer occurring in the liver is called liver cancer, and is one of the most prevalent cancers worldwide. In Korea, the mortality of liver cancer is very high (23 per 100,000 people), and approximately 10% of all deaths are associated with hepatitis, cirrhosis and liver cancer.

Liver cancer may be classified into metastatic liver cancer in which cancer in a different tissue spreads to the liver and hepatocellular carcinoma (HCC) in which cancer occurs in liver cells themselves, and since HCC accounts for 90% of all types of liver cancer, most cases refer to HCC.

Liver cancer is diagnosed by imaging methods such as ultrasound imaging, computed tomography (CT), magnetic resonance imaging (MRI) and hepatic angiography. Ultrasound imaging is highly influenced by sensitivity according to the size of liver cancer, and is used as a primary imaging method for detecting the onset of liver cancer.

Large liver cancer tissues of 5 cm or more show sensitivity of 75% or more, whereas small liver cancer tissues of less than 1 cm show sensitivity of approximately 42% (Gomaa et al., World J Gastro., 15:1301, 2009).

Computed tomography (CT) is an examination tool with the highest sensitivity, and the sensitivity of diagnosis may be almost 100% for liver cancer having a size of 2 cm or more, 93% for that having a size of 1 to 2 cm, and almost 60% for that having a size of 1 cm or less (Gomaa et al., World J Gastro., 15:1301, 2009).

However, this is a burdensome examination tool to be used as a routine screening test for the general public due to a relatively high cost.

In the case of liver cancer, the size of a tumor at the time of diagnosis is associated with prognosis, and to increase a patient's survival rate, liver cancer has to be detected early. Therefore, there is an urgent need for the development of diagnostic technology that can detect liver cancer early with high sensitivity.

Meanwhile, epigenetics is the field of researching the regulation of gene expression which occurs while the base sequence of DNA is not changed. Epigenetics studies the regulation of gene expression through epigenetic mutations such as DNA methylation, miRNA or histone acetylation, methylation, phosphorylation and ubiquitination.

The DNA methylation is the most studied epigenetic mutation. An epigenetic mutation may cause a gene function mutation and a change to tumor cells. Therefore, DNA methylation is associated with the expression (or suppression and induction) of regulatory genes for a disease in cells, and recently, methods for diagnosing cancer by measuring DNA methylation have been suggested.

DNA methylation mainly occurs at cytosine in the CpG island of the promoter site of a specific gene, and thereby, binding of a transcription factor is disturbed to block the expression of a specific gene (gene silencing), which is a main mechanism by which the function of the gene is lost without mutation in a coding sequence.

DNA methylation in a non-translation region such as an enhancer or a regulatory site, in addition to the promoter region of the gene, also works with the structural mutation of a chromosome and histone modification, and is known to become a causative mechanism for various diseases. This abnormal methylation/demethylation in the CpG island was reported in various diseases including cancer, and attempts to use it for diagnosis of various diseases by investigating the promoter methylation of a disease-related gene have been actively made.

The inventors selected a methylation site of a gene related to the onset of liver cancer, and according to an experiment for verifying this, intended to provide a method of diagnosing the risk or prognosis of liver cancer.

Throughout the specification, numerous papers and patent documents are referred and citations thereof are presented. The disclosures of cited papers and patent documents are incorporated herein by reference in their entirety to more clearly explain the level of the technical field to which the present invention belongs and the contents of the present invention.

### [Disclosure]

### [Technical Problem]

To solve the above-described problems of the prior art, the present invention is directed to providing a method of diagnosing the risk or prognosis of liver cancer by measuring a methylation level of a specimen using a specific probe which shows low methylation in normal tissue or blood but shows a high methylation level only in liver cancer tissue to find the risk of liver cancer early.

### [Technical Solution]

According to one aspect of the present invention, a method of assessing the prognosis or risk of liver cancer, which includes: (a) isolating DNA from a biological sample of a subject; and (b) measuring a methylation level of a CpG site at a location selected from the group consisting of the sequence from 25438725 to 25439276 on chromosome #2, the sequence from 95941906 to 95942979 on chromosome #12, the sequence from 134597357 to 134602649 on chromosome #10, the sequence from 144649774 to 144651774 on chromosome #8, the sequence from 47998899 to 47999517 on chromosome #1, the sequence from 26394102 to 26396102 on chromosome #2, the sequence from 104510870 to 104513913 on chromosome #8, the sequence from 98289604 to 98290404 on chromosome #8, the sequence from 63281034 to 63281347 on chromosome #2, the sequence from 67873388 to 67875600 on chromosome #8, the sequence from 76555366 to 76556079 on chromosome #4, the sequence from 63782394 to 63790471 on chromosome #1, the sequence from 7849945 to 7850439 on chromosome #5, the sequence from 39186777 to 39187968 on chromosome #2, and the sequence from 74207665 to 74208665 on chromosome #14 in the isolated DNA, is provided.

In one embodiment, the method may measure a methylation level at two or more CpG sites.

In one embodiment, the sequence from 25438725 to 25439276 on chromosome #2 may have the base sequence of SEQ ID NO: 1, the sequence from 95941906 to 95942979 on chromosome #12 may have the base sequence of SEQ ID NO: 2, the sequence from 134597357 to 134602649 on chromosome #10 may have the base sequence of SEQ ID NO: 3, the sequence from 144649774 to 144651774 on chromosome #8 may have the base sequence of SEQ ID NO: 4, the sequence from 47998899 to 47999517 on chromosome #1 may have the base sequence of SEQ ID NO: 5, the sequence from 26394102 to 26396102 on chromosome #2 may have the base sequence of SEQ ID NO: 6, the sequence from 104510870 to 104513913 on chromosome #8 may have the base sequence of SEQ ID NO: 7, the sequence from 98289604 to 98290404 on chromosome #8 may have the base sequence of SEQ ID NO: 8, the sequence from 63281034 to 63281347 on chromosome #2 may have the base sequence of SEQ ID NO: 9, the sequence from 67873388 to 67875600 on chromosome #8 may have the base sequence of SEQ ID NO: 10, the sequence from 76555366 to 76556079 on chromosome #4 may have the base sequence of SEQ ID NO: 11, the sequence from 63782394 to 63790471 on chromosome #1 may have the base sequence of SEQ ID NO: 12, the sequence from 7849945 to 7850439 on chromosome #5 may have the base sequence of SEQ ID NO: 13, the sequence from 39186777 to 39187968 on chromosome #2 may have the base sequence of SEQ ID NO: 14, and the sequence from 74207665 to 74208665 on chromosome #14 may have the base sequence of SEQ ID NO: 15.

In one embodiment, a CpG site of the sequence from 25438725 to 25439276 on chromosome #2 may be located at 25439110 of chromosome #2, a CpG site of the sequence from 95941906 to 95942979 on chromosome #12 may be located at 95941988 of chromosome #12, a CpG site of the sequence from 134597357 to 134602649 on chromosome #10 may be located at 134599823 of chromosome #10, a CpG site of the sequence from 144649774 to 144651774 on chromosome #8 may be located at 144651002 of chromosome #8, a CpG site of the sequence from 47998899 to 47999517 on chromosome #1 may be located at 47999163 of chromosome #1, a CpG site of the sequence from 26394102 to 26396102 on chromosome #2 may be located at 26395458 of chromosome #2, a CpG site of the sequence from 104510870 to 104513913 on chromosome #8 may be located at 104512877 of chromosome #8, a CpG site of the sequence from 98289604 to 98290404 on chromosome #8 may be located at 98290148 of chromosome #8, a CpG site of the sequence from 63281034 to 63281347 on chromosome #2 may be located at 63281139 of chromosome #2, a CpG site of the sequence from 67873388 to 67875600 on chromosome #8 may be located at 67874178 of chromosome #8, a CpG site of the sequence from 76555366 to 76556079 on chromosome #4 may be located at 76555832 of chromosome #4, a CpG site of the sequence from 63782394 to 63790471 on chromosome #1 may be located at 63789278 of chromosome #1, a CpG site of the sequence from 7849945 to 7850439 on chromosome #5 may be located at 7850070 of chromosome #5, a CpG site of the sequence from 39186777 to 39187968 on chromosome #2 may be located at 39187533 of chromosome #2, and a CpG site of the sequence from 74207665 to 74208665 on chromosome #14 may be located at 74208165 of chromosome #14.

In one embodiment, the biological sample may be one selected from the group consisting of tissue, cells, blood, plasma, stool and urine derived from a patient with suspected liver cancer or a subject diagnosed with liver cancer.

In one embodiment, step (b) may be performed by one method selected from the group consisting of PCR, methylation-specific PCR, real-time methylation-specific PCR, MethyLight PCR, MethyLight digital PCR, EpiTYPER, PCR using methylated DNA-specific binding protein, quantitative PCR, DNA chip assay, pyrosequencing and bisulfite sequencing.

In one embodiment, the method may further include (c) comparing the methylation level with a methylation level in a normal control.

According to another aspect of the present invention, a kit for diagnosing a risk of the onset of liver cancer, which includes a probe binding to a CpG site at a location selected from the group consisting of the sequence from 25438725 to 25439276 on chromosome #2, the sequence from 95941906 to 95942979 on chromosome #12, the sequence from 134597357 to 134602649 on chromosome #10, the sequence from 144649774 to 144651774 on chromosome #8, the sequence from 47998899 to 47999517 on chromosome #1, the sequence from 26394102 to 26396102 on chromosome #2, the sequence from 104510870 to 104513913 on chromosome #8, the sequence from 98289604 to 98290404 on chromosome #8, the sequence from 63281034 to 63281347 on chromosome #2, the sequence from 67873388 to 67875600 on chromosome #8, the sequence from 76555366 to 76556079 on chromosome #4, the sequence from 63782394 to 63790471 on chromosome #1, the sequence from 7849945 to 7850439 on chromosome #5, the sequence from 39186777 to 39187968 on chromosome #2, and the sequence from 74207665 to 74208665 on chromosome #14, is provided.

In one embodiment, the diagnostic kit may include two or more probes binding to CpG sites.

### [Advantageous Effects]

According to one aspect of the present invention, the possibility of the onset of liver cancer can be effectively predicted using a clinical specimen in which normal tissue is mixed by measuring methylation of a specific CpG site showing a different methylation level from most normal cells including blood as well as cancer and normal tissues.

It should be understood that the effect of the present invention is not limited to the above-described effects, and includes all effects that can be deduced from the configuration of the present invention described in the detailed description or claims of the present invention.

### [Description of Drawings]

FIG. 1 illustrates a liver cancer diagnostic marker selection pipeline of the present invention.

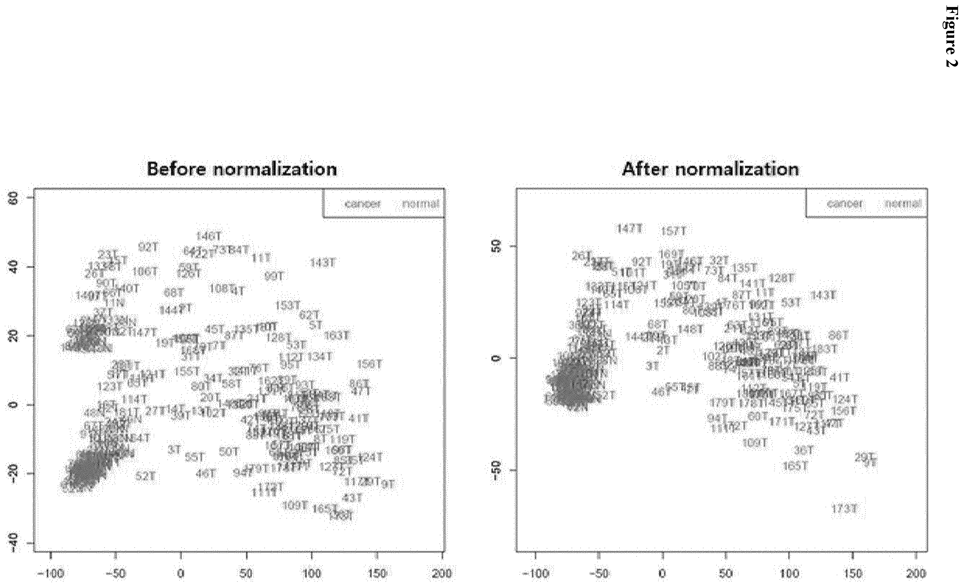

FIG. 2 is a set of graphs showing the distribution of liver cancer patients before (left) and after (right) normalization of DNA methylation data according to an exemplary embodiment of the present invention, respectively.
FIG. 3 is a heat map of differentially methylated probes (DMPs) which are hypermethylated in a liver cancer patient and hypomethylated in a normal person according to an exemplary embodiment of the present invention.
FIG. 4 is a set of heat maps showing the extent of methylation in a liver cancer sample, a normal liver sample and a blood sample for probes selected by heat maps. A red color indicates hypermethylation.
FIG. 5 shows the result of selecting a diagnostic marker according to an exemplary embodiment of the present invention through machine learning.
FIG. 6 is a set of heat maps confirming the extent of methylation of a diagnostic marker according to an exemplary embodiment of the present invention selected through machine learning in a liver cancer sample, a normal liver sample and a blood sample.
FIG. 7 shows a result of evaluating liver cancer diagnostic efficiency of a single probe according to one exemplary embodiment of the present invention. The liver cancer diagnostic efficiency per probe is represented as AUC.

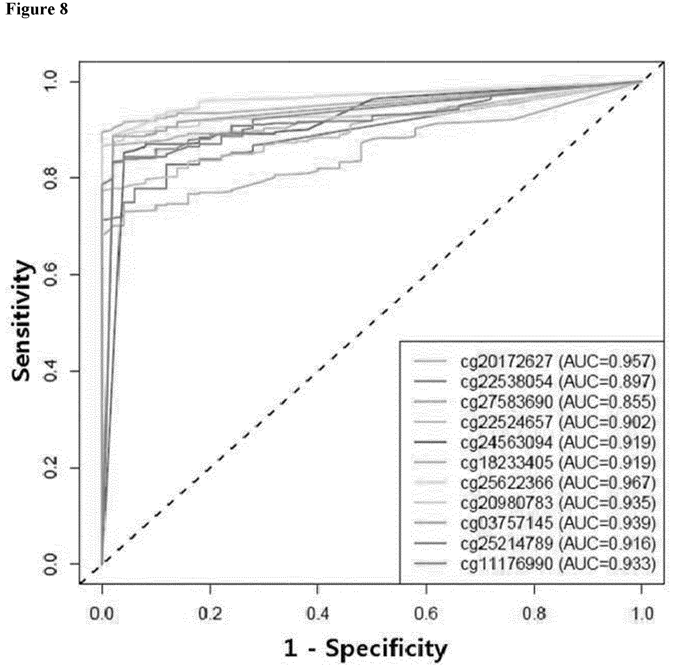

FIG. 8 shows a result of evaluating liver cancer diagnostic efficiency of a single probe according to an exemplary embodiment of the present invention in liver cancer data of the Cancer Genome Atlas (TCGA), which is a public DB. The liver cancer diagnostic efficiency per probe is represented as AUC.
FIG. 9 shows a result of confirming diagnostic efficiency according to the combination of probes (15 kinds) according to an exemplary embodiment of the present invention.
FIG. 10 is a set of heat maps showing the extent of methylation of probes selected according to an exemplary embodiment of the present invention through pyrosequencing. The x-axis represents 196 liver cancer samples in an independent cohort and normal liver samples corresponding thereto, and the y-axis represents CpG sites of a probe (yellow box) and near the probe.
FIG. 11 is a set of heat maps showing the extent of methylation of probes selected according to an exemplary embodiment of the present invention through an EpiTYPER experiment. The x-axis represents 184 liver cancer samples in an independent cohort and normal liver samples corresponding thereto, and the y-axis represents CpG sites of a probe (yellow box) and near the probe.

### [Modes of the Invention]

Hereinafter, the present invention will be described in detail with reference to the accompanying drawings. However, the present invention may be implemented in a variety of different forms, and is not limited to the embodiments described herein.

When one part "includes" a component, it means that, unless particularly stated otherwise, the part may further include another component, rather than excluding it.

Unless defined otherwise, the present invention may be carried out by conventional techniques frequently used in molecular biology, microbiology, protein purification, protein engineering, DNA sequencing, and the recombinant DNA field within the scope of those of ordinary skill in the art. The techniques are known to those of ordinary skill in the art, and described in many standardized textbooks and references.

Unless particularly defined otherwise, all technical and scientific terms used herein have the same meaning as generally understood by those of ordinary skill in the art.

Various scientific dictionaries, including the terms included herein, are well known and available in the art. Although any method and material which is similar or equivalent to those described herein are found in the execution or testing of the present invention, some methods and materials will be described. Depending on the context used by those of skill in the art, various methods and materials can be used, and thus the present invention is not limited to specific methodologies, protocols and reagents.

As used herein, singular forms include plural forms unless specifically stated otherwise. In addition, unless indicated otherwise, nucleic acids are written from left to right in the 5' to 3' direction, respectively, and the sequence of amino acids is written from left to right, in an amino to carboxyl direction. Hereinafter, the present invention will be described in further detail.

According to an aspect of the present invention, a method of assessing the prognosis or risk of liver cancer, which includes measuring a methylation level of one or more CpG sites, is provided.

The term "subject" may be a diagnostic target, which is a human, and the biological sample is a sample isolated from the subject to evaluate the risk of a liver cancer-related disease, including tissue, cells, blood, plasma, peritoneal fluid, synovial fluid, saliva, urine and stool, but the present invention is not limited thereto. Preferably, the biological sample may be blood, and specifically, plasma separated from blood.

In addition, the prognosis or risk of liver cancer may be diagnosed by individually analyzing a methylation level of the CpG site, but the accuracy of diagnosis is preferably enhanced by simultaneously analyzing two, three or four or more CpG sites.

The diagnosis is for determining the susceptibility of a subject for a specific disease or disorder, and preferably, determining whether a subject currently has liver cancer or determining the prognosis of a subject with liver cancer, and may include therametrics.

The "methylation" refers to attachment of a methyl group to a base constituting DNA. Preferably, methylation refers to methylation occurring at cytosine of a specific CpG site of a specific gene.

The "methylated state" refers to the presence or absence of 5-methyl cytosine of one or more CpG dinucleotides in a DNA base sequence. The "methylation level" refers to, for example, a level of methylation present in the DNA base sequences of target DNA-methylated genes in all genomic regions and some non-genomic regions.

The methylation level may be measured by one method selected from the group consisting of PCR, methylation-specific PCR, real-time methylation-specific PCR, MethyLight PCR, MethyLight digital PCR, EpiTYPER, PCR using a methylated DNA-specific binding protein, quantitative PCR, a DNA chip assay, pyrosequencing and bisulfite sequencing, but the present invention is not limited thereto.

The extent of methylation may be identified by a microarray. The microarray may be performed using a probe immobilized on a solid surface. The probe may include a sequence complementary to a sequence of 10 to 100 consecutive nucleotides in each gene containing an SNP.

The CpG site refers to a CpG site present in DNA of the gene. DNA of the gene is the concept including a series of all structural units that are required for expression and operably linked to each other, and includes, for example, a promoter region, a protein coding region (open reading frame, ORF) and a terminator region.

Therefore, the CpG site of the gene may be present in the promoter region, protein coding region (OFR) or terminator region of a corresponding gene. As a preferable example, the CpG site of the gene may be a CpG site present in the promoter region of the gene.

The CpG site may be present in one or more base sequences selected from the group consisting of the sequence from 25438725 to 25439276 on chromosome #2, the sequence from 95941906 to 95942979 on chromosome #12, the sequence from 134597357 to 134602649 on chromosome #10, the sequence from 144649774 to 144651774 on chromosome #8, the sequence from 47998899 to 47999517 on chromosome #1, the sequence from 26394102 to 26396102 on chromosome #2, the sequence from 104510870 to 104513913 on chromosome #8, the sequence from 98289604 to 98290404 on chromosome #8, the sequence from 63281034 to 63281347 on chromosome #2, the sequence from 67873388 to 67875600 on chromosome #8, the sequence from 76555366 to 76556079 on chromosome #4, the sequence from 63782394 to 63790471 on chromosome #1, the sequence from 7849945 to 7850439 on chromosome #5, the sequence from 39186777 to 39187968 on chromosome #2, and the sequence from 74207665 to 74208665 on chromosome #14.

The sequence from 25438725 to 25439276 on chromosome #2 may have the base sequence of SEQ ID NO: 1, the sequence from 95941906 to 95942979 on chromosome #12 may have the base sequence of SEQ ID NO: 2, the sequence from 134597357 to 134602649 on chromosome #10 may have the base sequence of SEQ ID NO: 3, the sequence from 144649774 to 144651774 on chromosome #8 may have the base sequence of SEQ ID NO: 4, the sequence from 47998899 to 47999517 on chromosome #1 may have the base sequence of SEQ ID NO: 5, the sequence from 26394102 to 26396102 on chromosome #2 may have the base sequence of SEQ ID NO: 6, the sequence from 104510870 to 104513913 on chromosome #8 may have the base sequence of SEQ ID NO: 7, the sequence from 98289604 to 98290404 on chromosome #8 may have the base sequence of SEQ ID NO: 8, the sequence from 63281034 to 63281347 on chromosome #2 may have the base sequence of SEQ ID NO: 9, the sequence from 67873388 to 67875600 on chromosome #8 may have the base sequence of SEQ ID NO: 10, the sequence from 76555366 to 76556079 on chromosome #4 may have the base sequence of SEQ ID NO: 11, the sequence from 63782394 to 63790471 on chromosome #1 may have the base sequence of SEQ ID NO: 12, the sequence from 7849945 to 7850439 on chromosome #5 may have the base sequence of SEQ ID NO: 13, the sequence from 39186777 to 39187968 on chromosome #2 may have the base sequence of SEQ ID NO: 14, and the sequence from 74207665 to 74208665 on chromosome #14 may have the base sequence of SEQ ID NO: 15.

The CpG site of the sequence from 25438725 to 25439276 on chromosome #2 may be located at 25439110 of chromosome #2, the CpG site of the sequence from 95941906 to 95942979 on chromosome #12 may be located at 95941988 of chromosome #12, the CpG site of the sequence from 134597357 to 134602649 on chromosome #10 may be located at 134599823 of chromosome #10, the CpG site of the sequence from 144649774 to 144651774 on chromosome #8 may be located at 144651002 of chromosome #8, the CpG site of the sequence from 47998899 to 47999517 on chromosome #1 may be located at 47999163 of chromosome #1, the CpG site of the sequence from 26394102 to 26396102 on chromosome #2 may be located at 26395458 of chromosome #2, the CpG site of the sequence from 104510870 to 104513913 on chromosome #8 may be located at 104512877 of chromosome #8, the CpG site of the sequence from 98289604 to 98290404 on chromosome #8 may be located at 98290148 of chromosome #8, the CpG site of the sequence from 63281034 to 63281347 on chromosome #2 may be located at 63281139 of chromosome #2, the CpG site of the sequence from 67873388 to 67875600 on chromosome #8 may be located at 67874178 of chromosome #8, the CpG site of the sequence from 76555366 to 76556079 on chromosome #4 may be located at 76555832 of chromosome #4, the CpG site of the sequence from 63782394 to 63790471 on chromosome #1 may be located at 63789278 of chromosome #1, the CpG site of the sequence from 7849945 to 7850439 on chromosome #5 may be located at 7850070 of chromosome #5, the CpG site of the sequence from 39186777 to 39187968 on chromosome #2 may be located at 39187533 of chromosome #2, and the CpG site of the sequence from 74207665 to 74208665 on chromosome #14 may be located at 74208165 of chromosome #14.

According to another aspect of the present invention, a kit for diagnosing a risk of the onset of liver cancer, which includes a probe binding to a CpG site at a location selected from the group consisting of the sequence from 25438725 to 25439276 on chromosome #2, the sequence from 95941906 to 95942979 on chromosome #12, the sequence from 134597357 to 134602649 on chromosome #10, the sequence from 144649774 to 144651774 on chromosome #8, the sequence from 47998899 to 47999517 on chromosome #1, the sequence from 26394102 to 26396102 on chromosome #2, the sequence from 104510870 to 104513913 on chromosome #8, the sequence from 98289604 to 98290404 on chromosome #8, the sequence from 63281034 to 63281347 on chromosome #2, the sequence from 67873388 to 67875600 on chromosome #8, the sequence from 76555366 to 76556079 on chromosome #4, the sequence from 63782394 to 63790471 on chromosome #1, the sequence from 7849945 to 7850439 on chromosome #5, the sequence from 39186777 to 39187968 on chromosome #2, and the sequence from 74207665 to 74208665 on chromosome #14, is provided.

The probe may be used as a hybridizable array element and immobilized on a substrate.

The substrate may be a suitable rigid or semi-rigid support, and include, for example, a membrane, a filter, a chip, a slide, a wafer, a fiber, a magnetic or nonmagnetic bead, a gel, tubing, a plate, a polymer, a microparticle, and a capillary tube. The hybridizable array element may be arranged on the substrate and immobilized thereon.

The immobilization may be performed by a chemical binding method or a covalent bonding method such as UV. For example, the hybridizable array element may be bound to a glass surface modified to include an epoxy compound or an aldehyde group, and bound to a polylysine-coated surface by UV. In addition, the hybridizable array element may be bound to the substrate by linkers (e.g., an ethylene glycol oligomer and a diamine).

Sample DNA applied to the microarray may be labeled, and hybridized with an array element. Hybridization conditions may be changed in various ways, and the detection and analysis of the extent of hybridization may be carried out in various ways according to a labeling substance.

Labeling of the probe may provide a signal that allows the detection of hybridization, and may be linked to an oligonucleotide.

The label may include a fluorophore (e.g., fluorescein, phycoerythrin, rhodamine, lissamine, Cy3 or Cy5 (Pharmacia)), a chromophore, a chemiluminophore, a magnetic particle, a radioisotope (P32 or S35), a mass label, an electron dense particle, an enzyme (alkaline phosphatase or horseradish peroxidase), a cofactor, a substrate for an enzyme, a heavy metal (e.g., gold), an antibody, streptavidin, biotin, digoxigenin, or a hapten with a specific binding partner such as a chelating group, but the present invention is not limited thereto.

The label may be labeled by various methods conventionally performed in the art, for example, a nick translation method, a random priming method (Multiprime DNA labelling systems booklet, "Amersham" (1989)) and a kination method (Maxam & Gilbert, Methods in Enzymology, 65:499(1986)).

The label may provide a signal that can be detected by fluorescence, radioactivity, colorimetry, gravimetry, X-ray diffraction or absorption, magnetism, enzymatic activity, mass spectrometry, binding affinity, hybridization radiofrequency or nanocrystals.

The nucleic acid sample to be analyzed may be prepared using mRNA obtained from various biosamples. Instead of the probe, cDNA to be analyzed may be labeled to perform hybridization-based analysis.

When the probe is used, the probe may be hybridized with a cDNA molecule. The suitable hybridization conditions may be determined in a series of steps by an optimization procedure. The procedure may be performed in a series of steps by those of ordinary skill in the art to establish a protocol to be used in a laboratory.

For example, conditions such as a temperature, the concentration of a component, hybridization and washing time, components of a buffer solution, pH and ionic strength depend on various parameters such as a probe length, a GC content and a target nucleotide sequence. For detailed conditions for the hybridization, Joseph Sambrook et al., Molecular Cloning, A Laboratory Manual, Cold Spring Harbor Laboratory Press, Cold Spring Harbor, N.Y. (2001); and M.L.M. Anderson, Nucleic Acid Hybridization, Springer-Verlag New York Inc. N.Y. (1999) may be referenced.

For example, high stringent conditions among the stringent conditions may refer to hybridization under conditions of 0.5 M NaHPO₄, 7% sodium dodecyl sulfate (SDS), and 1 mM EDTA at 65 °C, and washing in 0.1 x standard saline citrate (SSC)/0.1% SDS at 68 °C. Alternatively, the high stringent condition may refer to washing in 6x SSC/0.05% sodium pyrophosphate at 48 °C, and a low stringent condition may refer to washing in 0.2 x SSC/0.1% SDS at 42 °C.

After the hybridization, a hybridization signal emitted by hybridization may be detected. For example, when the probe is labeled with an enzyme, hybridization may be confirmed by reacting a substrate of the enzyme with a hybridization reaction product.

The enzyme and substrate may be a peroxidase (e.g., horseradish peroxidase) and chloronaphthol, aminoethyl carbazole, diaminobenzidine, D-luciferin, bis-N-methylacridinium nitrate (Lucigenin), resorufin benzyl ether, luminol, the Amplex Red reagent (10-acetyl-3,7-dihydroxyphenoxazine), HYR (p-phenylenediamine-HCl and pyrocatechol), tetramethylbenzidine (TMB), 2,2'-azine-di[3-ethylbenzthiazoline sulfonate] (ABTS), o-phenylenediamine (OPD) or naphthol/pyronine; alkaline phosphatase and bromochloroindolyl phosphate (BCIP), nitroblue tetrazolium (NBT) or naphthol-AS-Bl-phosphate and an ECF substrate; and glucose oxidase and nitroblue tetrazolium (t-NBT) or phenazine methosulfate (m-PMS).

When the probe is labeled with gold particles, it may be detected by a silver staining method using silver nitrate.

The method of assessing the prognosis or risk of liver cancer may be assessing the possibility of diagnosing liver cancer by various statistical methods. In one embodiment, a statistical method may be a machine learning method, and Maxwell W. Libbrecht, 2015, Nature Reviews Genetics 16: 321-332 may be referenced.

The machine learning is a field of artificial intelligence that has evolved from the study of pattern recognition and computer learning theory. Machine learning is technology of studying and establishing a system for experimental data-based learning, prediction and enhancement of performance by itself and algorithms therefor. The algorithms of machine learning are methods of constructing a specific model to elicit predictions or determinations based on input data, rather than carrying out strictly determined static program commands.

Hereinafter, the present invention will be described in further detail with reference to examples.

### Example 1. Selection of differentially methylated probe (DMP) associated with onset of liver cancer

### Samples

Liver cancer samples were obtained from 184 liver cancer patients in the Seoul National University Hospital to select a DNA methylation region associated with the onset of liver cancer. Normal tissue corresponding to liver cancer tissue was used as a normal control.

Genomic DNA was extracted from each sample using a column-based DNA extraction method (PureLink™ Genomic DNA Mini Kit, Invitrogen) and a bead-type DNA extraction method (MagListo™5M Genomic DNA Extraction Kit, Bioneer). The extracted genomic DNA was quantified using NanoDrop, and a DNA state was identified by confirming degradation through electrophoresis in 1.5% agarose gel.

### Bisulfite treatment

The extent of methylation may be measured in such a way that, after genomic DNA is treated with bisulfite, when cytosine of the 5'-CpG-3' site of the DNA base sequence is methylated, the cytosine may remain the same, whereas when non-methylated, the cytosine is changed to uracil.

Therefore, to distinguish methylated cytosine and non-methylated cytosine, genomic DNA was treated with bisulfite. 700 ng of genomic DNA was treated using an EZ DNA Methylation Kit (Zymoresearch Inc.) according to the manufacturer's manual, and the bisulfite-treated DNA prepared thereby was dissolved in M-elution buffer and then stored at -80 °C before use.

The bisulfite-treated DNA was used within a month.

### DNA methylation microarray

A DNA methylation microarray was performed using Infinium® Human Methylation 850K BeadChip.

Using Illumina Infinium Methylation EPIC BeadChip kits (Illumina, Inc., San Diego, CA) according to the manufacturer's manual, bisulfite-treated DNA was amplified, subjected to fragmentation, precipitation and resuspension, and hybridized with a BeadChip.

After washing, the BeadChip was scanned using an Illumina iScan scanner.

Among R packages, according to the manual in a package using a minfi package, the quality control of data was performed. Only for samples passing the standards of quality control, a β value, which is a value obtained by digitizing the idat file of raw data showing the extent of methylation in color, was calculated.

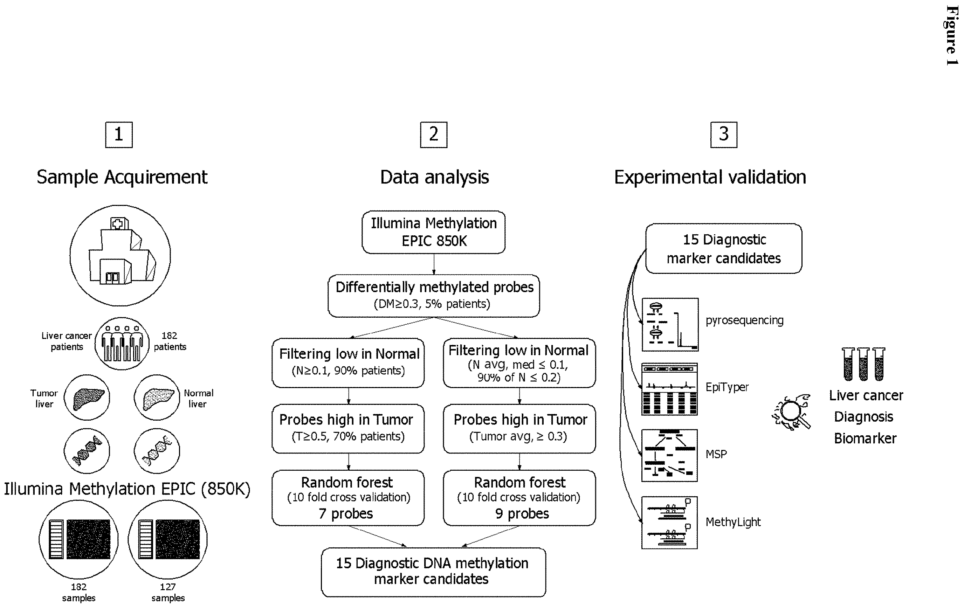

The extent of DNA methylation was represented as a β value of 0 to 1, where the β value of 0 represents that a corresponding CpG site is completely non-methylated, and the β value of 1 represents that a corresponding CpG site is completely methylated. The calculated results were normalized and corrected. All statistics were performed in the R statistical environment (v.3.3.2 or higher; FIG. 1).

### Example 2. Selection of diagnostic marker candidate

Referring to FIG. 1, DNA was extracted from 182 liver cancer samples and normal liver samples corresponding thereto, and subjected to an Infinium Methylation EPIC BeadChip assay.

Methylation data was analyzed with a self-constructed pipeline. Probes that exhibited low methylation levels in the normal samples and high methylation levels in the tumor samples were selected.

First, a DMP which had a methylation difference between a normal sample and a cancer sample was selected.

Seven probes which exhibited very low methylation levels in the normal samples and very high methylation levels of 50% or more in 70% or more of the cancer patients were selected, and efficiency was verified by a machine learning method (FIG. 1, dark blue).

Probes which exhibited very low methylation levels of 10% or less in the normal samples and high methylation levels of 30% or more on average in the liver cancer patients were selected, and the top nine probes that effectively distinguish liver cancer/normal liver samples were selected by machine learning (FIG. 1, brown).

The finally selected 15 (1 duplicate) liver cancer diagnostic marker candidates were verified by various experiments.

### Example 3. Selection of probes by heat map

As a result of investigating DNA methylation of 182 liver cancer samples and 127 normal samples, 100,053 DMP with 30% or more hypermethylation were selected from 5% or more of the liver cancer samples.

Among DMPs having a difference between normal/cancer samples, 13,078 probes exhibiting very low methylation levels of 10% or less were selected from normal samples such that blood biopsy was possible.

Among the selected probes, seven 50% or more-hypermethylated probes were selected from 70% or more of the cancer patients (Table 1).

**[Table 1]**

| **Division** | **Probe ID** | **50% or more hypermethylated liver cancer ratio (%)** |
|---|---|---|
| Probe 1 | cg20172627 | 78.16 |
| Probe 2 | cg22538054 | 77.59 |
| Probe 3 | cg27583690 | 74.14 |
| Probe 4 | cg19951303 | 72.99 |
| Probe 5 | cg22524657 | 71.84 |
| Probe 6 | cg24563094 | 70.11 |
| Probe 7 | cg25744484 | 70.11 |

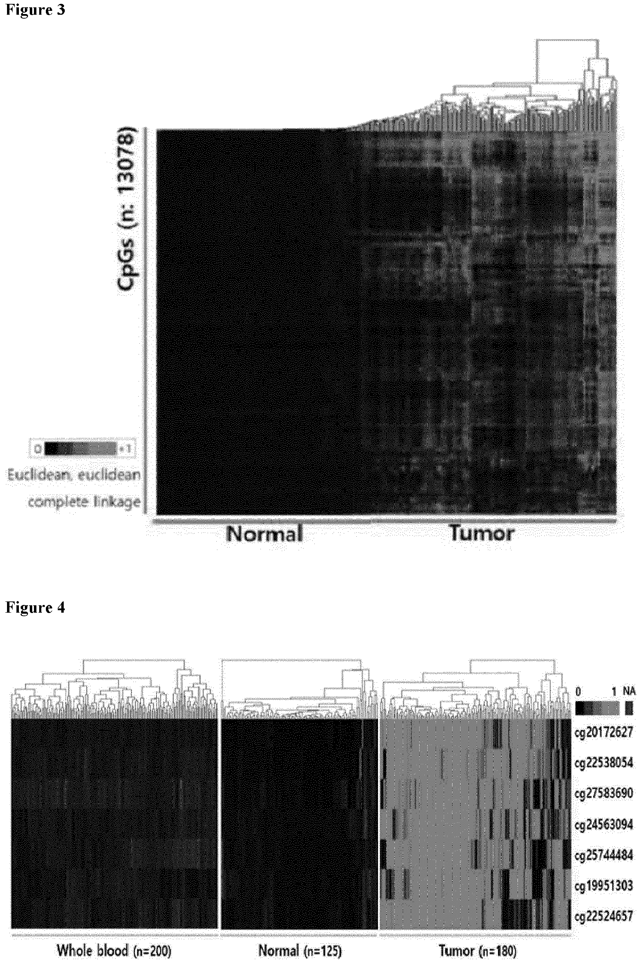

Heat maps were made to confirm liver cancer patient-specific methylation levels of the selected seven probes (FIG. 4).

### Example 4. Selection of probe by machine learning

Among DMPs showing a difference between normal/cancer samples, probes that exhibit very low methylation levels in normal samples and high methylation levels of 30% or more on average in liver cancer patients were selected.

The top nine probes effectively distinguishing liver cancer/normal liver samples were selected through machine learning using the previously-selected probes.

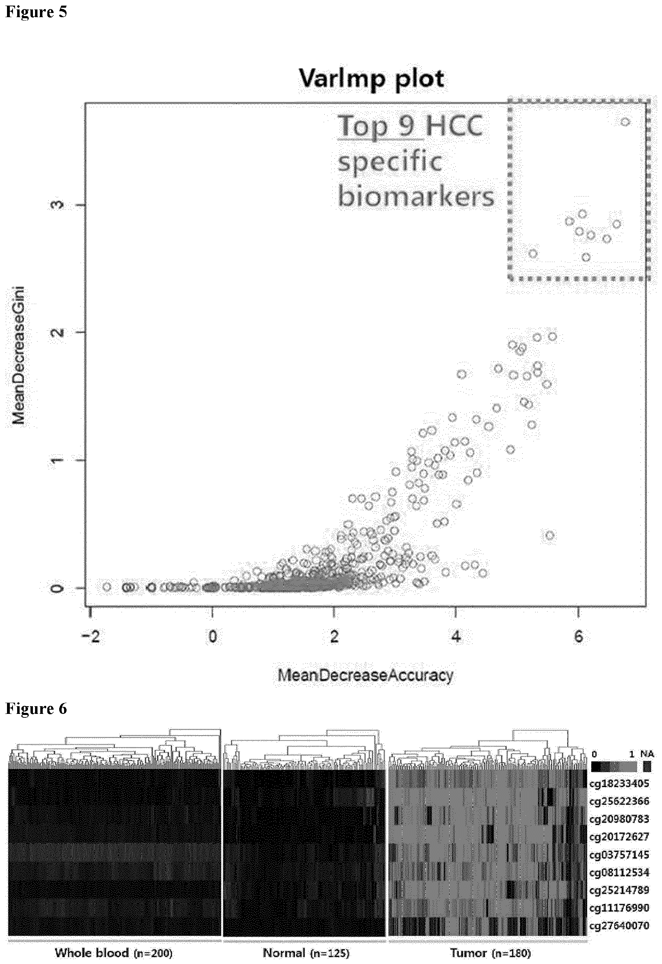

Referring to FIG. 5, a blue dot represents one probe, and the top nine probes were selected in order of importance (x- and y-axes).

The x-axis represents the accuracy of each probe in a model constructed by machine learning, and the y-axis represents the purity of each probe in a model constructed by machine learning.

Heat maps were made to confirm the extent of methylation of the 9 probes selected by machine learning in 200 whole blood samples, 125 normal samples and 180 liver cancer samples (FIG. 6).

Information on the 15 probes finally selected by the methods described in Examples 4 and 5 is shown in Table 2 below.

**[Table 2]**

| **SEQ ID NO:** | **Probe ID** | **Selection method** | **CpG location** | | | **CGI region** |
|---|---|---|---|---|---|---|
| | | | **Chromosomestart** | | **End** | |
| 1 | cg20172627 | heatmap | chr2 | 25438725 | 25439276 | Island |
| 2 | cg22538054 | heatmap | chr12 | 95941906 | 95942979 | Island |
| 3 | cg27583690 | heatmap | chr10 | 134597357 | 134602649 | Island |
| 4 | cg19951303 | heatmap | chr8 | 144649774 | 144651774 | N_Shelf |
| 5 | cg22524657 | heatmap | chr1 | 47998899 | 47999517 | Island |
| 6 | cg24563094 | heatmap | chr2 | 26394102 | 26396102 | N_Shore |
| 7 | cg25744484 | heatmap | chr8 | 104510870 | 104513913 | Island |
| 8 | cg18233405 | machine learning | chr8 | 98289604 | 98290404 | Island |
| 9 | cg25622366 | machine learning | chr2 | 63281034 | 63281347 | Island |
| 10 | cg20980783 | machine learning | chr8 | 67873388 | 67875600 | Island |
| 1 | cg20172627 | machine learning | chr2 | 25438725 | 25439276 | Island |
| 11 | cg03757145 | machine learning | chr4 | 76555366 | 76556079 | Island |
| 12 | cg08112534 | machine learning | chr1 | 63782394 | 63790471 | Island |
| 13 | cg25214789 | machine learning | chr5 | 7849945 | 7850439 | Island |
| 14 | cg11176990 | machine learning | chr2 | 39186777 | 39187968 | Island |
| 15 | cg27640070 | machine learning | chr14 | 74207665 | 74208665 | - |

### Example 5. Evaluation of liver cancer diagnosis efficiency of single probes

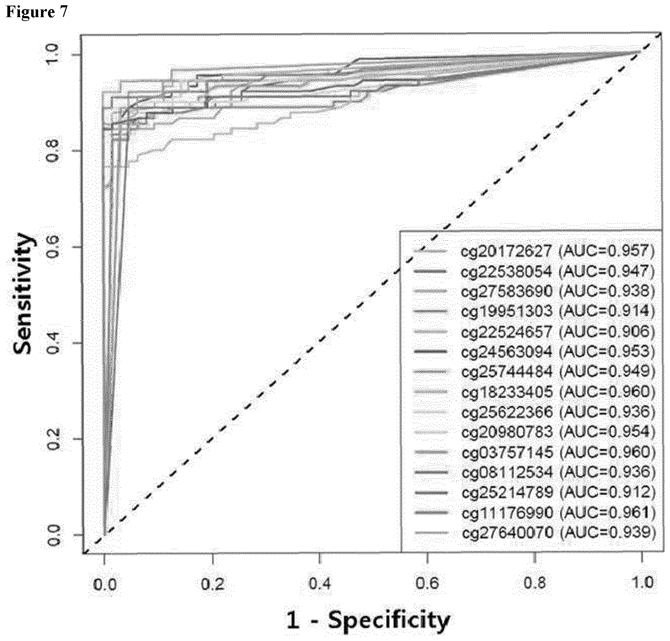

The liver cancer diagnostic efficiency of the selected 15 probes was evaluated (FIG. 7).

FIG. 7 shows the liver cancer diagnostic efficiency per probe, represented as AUC.

Liver cancer diagnostic efficiency (AUC; area under the curve) was confirmed only using 15 probes, and the results are shown in Table 3 below.

**[Table 3]**

| **SEQ ID NO:** | **probe ID** | **Selection method** | **Acuu.** | **Sen.** | **Spe.** | **AUC** |
|---|---|---|---|---|---|---|
| 1 | cg20172627 | heatmap | 0.908 | 0.922 | 0.887 | 0.957 |
| 2 | cg22538054 | heatmap | 0.888 | 0.878 | 0.903 | 0.947 |
| 3 | cg27583690 | heatmap | 0.863 | 0.856 | 0.873 | 0.938 |
| 4 | cg19951303 | heatmap | 0.837 | 0.889 | 0.762 | 0.914 |
| 5 | cg22524657 | heatmap | 0.811 | 0.822 | 0.794 | 0.906 |
| 6 | cg24563094 | heatmap | 0.889 | 0.922 | 0.841 | 0.953 |
| 7 | cg25744484 | heatmap | 0.882 | 0.889 | 0.871 | 0.949 |
| 8 | cg18233405 | machine learning | 0.948 | 0.944 | 0.952 | 0.960 |
| 9 | cg25622366 | machine learning | 0.908 | 0.889 | 0.936 | 0.936 |
| 10 | cg20980783 | machine learning | 0.888 | 0.878 | 0.903 | 0.954 |
| 11 | cg03757145 | machine learning | 0.909 | 0.922 | 0.889 | 0.960 |
| 12 | cg08112534 | machine learning | 0.855 | 0.889 | 0.807 | 0.936 |
| 13 | cg25214789 | machine learning | 0.863 | 0.889 | 0.825 | 0.912 |
| 14 | cg11176990 | machine learning | 0.882 | 0.922 | 0.823 | 0.961 |
| 15 | cg27640070 | machine learning | 0.895 | 0.900 | 0.889 | 0.939 |

In addition, from the public DB, the liver cancer diagnostic efficiency of a single probe was verified (FIG. 7). FIG. 7 shows the liver cancer diagnostic efficiency per probe, represented as AUC.

The result of verifying the efficiency of single probes using TCGA LIHC methylation data (450K) is shown in Table 4 below.

The region marked with gray(-) indicates a probe which is not found in an Infinium Methylation 450K BeadChip, and found only in an Infinium Methylation EPIC BeadChip (850K).

**[Table 4]**

| **SEQ ID NO:** | **Probe ID** | **Selection method** | **Acuu.** | **Sen.** | **Spe.** | **AUC** |
|---|---|---|---|---|---|---|
| 1 | cg20172627 | heatmap | 0.916 | 0.918 | 0.900 | 0.957 |
| 2 | cg22538054 | heatmap | 0.797 | 0.786 | 0.880 | 0.897 |
| 3 | cg27583690 | heatmap | 0.764 | 0.754 | 0.840 | 0.855 |
| 4 | cg19951303 | heatmap | - | - | - | - |
| 5 | cg22524657 | heatmap | 0.816 | 0.815 | 0.820 | 0.902 |
| 6 | cg24563094 | heatmap | 0.870 | 0.876 | 0.820 | 0.919 |
| 7 | cg25744484 | heatmap | - | - | - | - |
| 8 | cg18233405 | machine learning | 0.893 | 0.902 | 0.820 | 0.919 |
| 9 | cg25622366 | machine learning | 0.888 | 0.879 | 0.960 | 0.967 |
| 10 | cg20980783 | machine learning | 0.897 | 0.897 | 0.900 | 0.935 |
| 11 | cg03757145 | machine learning | 0.890 | 0.879 | 0.980 | 0.939 |
| 12 | cg08112534 | machine learning | - | - | - | - |
| 13 | cg25214789 | machine learning | 0.881 | 0.887 | 0.840 | 0.916 |
| 14 | cg11176990 | machine learning | 0.846 | 0.852 | 0.800 | 0.933 |
| 15 | cg27640070 | machine learning | - | - | - | - |

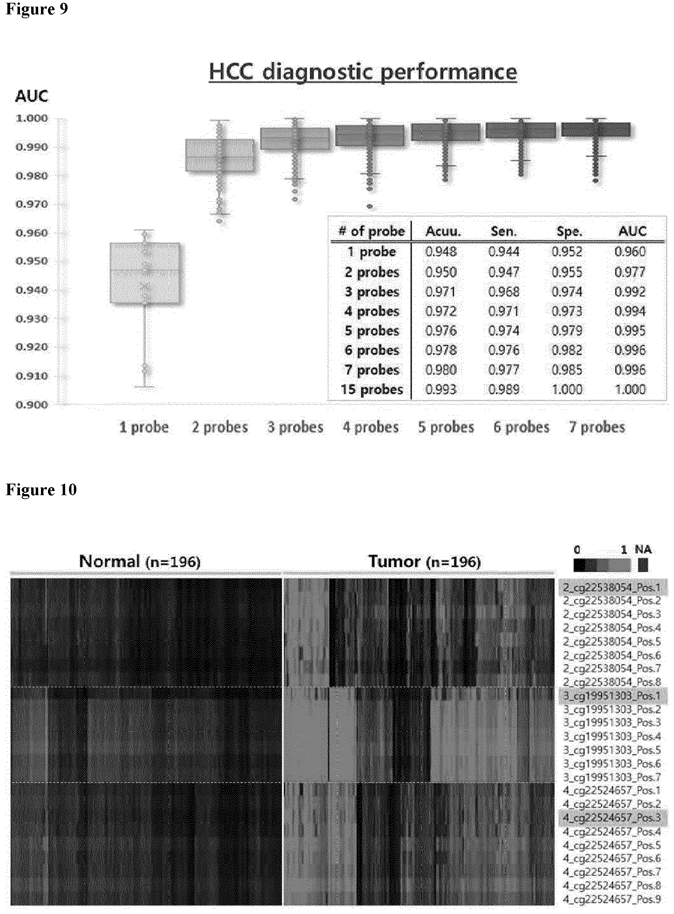

In addition, to analyze the liver cancer diagnostic efficiency of 15 panel probes, the liver cancer diagnostic efficiency (AUC) was confirmed by combining 15 probes (FIG. 9). FIG. 9 shows the confusion matrix result of training data and validation data obtained by machine learning with 15 probes (secondary cross validation).

To prevent data bias, secondary cross validation for randomly dividing data into two sets was performed 10 times, and thus the data was classified into a testing set and a training set.

Based on the data classified as the training set, normal and liver cancer patterns were learned, and a liver cancer-specific diagnosis model according thereto was constructed.

Table 5 shows an error matrix of the training set.

**[Table 5]**

| Input value | Determined as normal | Determined as liver cancer | Error rate |
|---|---|---|---|
| Normal | 62 | 1 | 0.159 |
| Liver cancer | 3 | 87 | 0.333 |

The test set was diagnosed based on the liver cancer-specific diagnosis model constructed with the training set, thereby confirming liver cancer diagnostic efficiency (Table 6).

**[Table 6]**

| Sample | Determined as normal | Determined as liver cancer |
|---|---|---|
| Normal | 61 | 0 |
| Liver cancer | 1 | 90 |

Referring to Tables 5 and 6, a liver cancer-specific diagnosis model was able to be constructed with the 15 probes selected based on machine learning, and diagnostic efficiency was evaluated at a very high level.

### Example 6. Evaluation of liver cancer diagnosis efficiency using several probes

To determine the minimum number of probes having the maximum efficiency among the 15 probes based on the liver cancer-specific diagnosis model, efficiency per the number of probes was measured (FIG. 9).

FIG. 9 shows the result obtained by machine learning with possible probe combinations (secondary cross validation). The x-axis represents the number of probes, and the y-axis represents AUC (diagnostic efficiency).

Referring to FIG. 9, when the number of probes is 3 or more, since the diagnostic efficiency approaches 99% or more, very accurate diagnosis data may be provided.

Accordingly, compared with the use of a single probe, the use of several probes may significantly improve diagnostic accuracy.

### Example 7. Analysis of methylation of CpG island including probe by pyrosequencing

To measure the extent of methylation at a CpG site to which one of the selected probes is bound, pyrosequencing was performed.

Pyrosequencing uses a pyrophosphate (PPi) emitted by addition of a nucleotide. PPi is converted into ATP by ATP sulfurylase in the presence of adenosine 5'-phosphate.

Luciferase is used to convert luciferin into oxyluciferin by ATP, and this reaction produces light that is able to be detected and analyzed.

The extent of methylation at CpG sites of the selected probes are shown by heat maps (FIG. 10).

As a result, it was confirmed that the methylation level was low in Normal, and high in Tumor, and the extent of methylation at CpG sites of the selected probes and their surroundings was similar.

### Example 8. Analysis of methylation of CpG island including probe by EpiTYPER

To verify data, methylation states of the top three probes among the selected probes were quantitatively analyzed using an EpiTYPER™ assay (Sequenom, San Diego, CA).

After PCR amplification, amplicons transcribed *in vitro* were treated with shrimp alkaline phosphatase, cleaved with RNaseA, and to determine the methylation state, subjected to MALDI-TOF Mass Spectrometry.

The result was analyzed using EpiTYPER™ ver. 1.0 software.

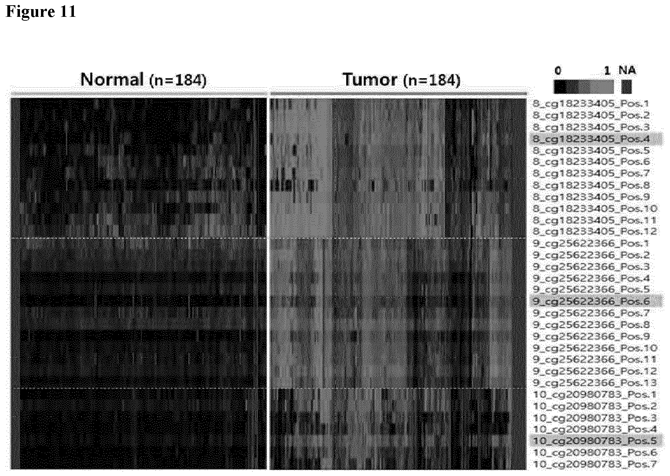

Validation was performed for the three selected probes by EpiTYPER. The extent of methylation at CpG sites of the selected probes and their surroundings was confirmed by heat maps (FIG. 11).

Referring to FIG. 11, it was confirmed that the methylation level was low in Normal and high in Tumor, and the extent of methylation at CpG sites of the selected probes and their surroundings was similar.

Accordingly, methylation levels of all of the CpG islands including a CPG probe can be used for diagnosing the prognosis and risk of cancer as described above.

It should be understood by those of ordinary skill in the art that the above description of the present invention are exemplary, and the example embodiments disclosed herein can be easily modified into other specific forms without changing the technical spirit or essential features of the present invention. Therefore, it should be interpreted that the example embodiments described above are exemplary in all aspects, and are not limitative. For example, each component described as a single unit may be distributed and implemented, and components described as being distributed may also be implemented in combined form.

The scope of the present invention is defined by the appended claims and encompasses all modifications and alterations derived from meanings, the scope and equivalents of the appended claims.

## Claims

1. A method of assessing the prognosis or risk of liver cancer, comprising:
(a) isolating DNA from a biological sample of a subject; and
(b) measuring a methylation level of a CpG site at a location selected from the group consisting of the sequence from 25438725 to 25439276 on chromosome #2, the sequence from 95941906 to 95942979 on chromosome #12, the sequence from 134597357 to 134602649 on chromosome #10, the sequence from 144649774 to 144651774 on chromosome #8, the sequence from 47998899 to 47999517 on chromosome #1, the sequence from 26394102 to 26396102 on chromosome #2, the sequence from 104510870 to 104513913 on chromosome #8, the sequence from 98289604 to 98290404 on chromosome #8, the sequence from 63281034 to 63281347 on chromosome #2, the sequence from 67873388 to 67875600 on chromosome #8, the sequence from 76555366 to 76556079 on chromosome #4, the sequence from 63782394 to 63790471 on chromosome #1, the sequence from 7849945 to 7850439 on chromosome #5, the sequence from 39186777 to 39187968 on chromosome #2, and the sequence from 74207665 to 74208665 on chromosome #14 in the isolated DNA.

2. The method of claim 1, wherein levels of methylation at 2 or more CpG sites are measured.

3. The method of claim 1, wherein the sequence from 25438725 to 25439276 on chromosome #2 has the base sequence of SEQ ID NO: 1, the sequence from 95941906 to 95942979 on chromosome #12 has the base sequence of SEQ ID NO: 2, the sequence from 134597357 to 134602649 on chromosome #10 has the base sequence of SEQ ID NO: 3, the sequence from 144649774 to 144651774 on chromosome #8 has the base sequence of SEQ ID NO: 4, the sequence from 47998899 to 47999517 on chromosome #1 has the base sequence of SEQ ID NO: 5, the sequence from 26394102 to 26396102 on chromosome #2 has the base sequence of SEQ ID NO: 6, the sequence from 104510870 to 104513913 on chromosome #8 has the base sequence of SEQ ID NO: 7, the sequence from 98289604 to 98290404 on chromosome #8 has the base sequence of SEQ ID NO: 8, the sequence from 63281034 to 63281347 on chromosome #2 has the base sequence of SEQ ID NO: 9, the sequence from 67873388 to 67875600 on chromosome #8 has the base sequence of SEQ ID NO: 10, the sequence from 76555366 to 76556079 on chromosome #4 has the base sequence of SEQ ID NO: 11, the sequence from 63782394 to 63790471 on chromosome #1 has the base sequence of SEQ ID NO: 12, the sequence from 7849945 to 7850439 on chromosome #5 has the base sequence of SEQ ID NO: 13, the sequence from 39186777 to 39187968 on chromosome #2 has the base sequence of SEQ ID NO: 14, and the sequence from 74207665 to 74208665 on chromosome #14 has the base sequence of SEQ ID NO: 15.

4. The method of claim 1, wherein a CpG site of the sequence from 25438725 to 25439276 on chromosome #2 is located at 25439110 of chromosome #2, a CpG site of the sequence from 95941906 to 95942979 on chromosome #12 is located at 95941988 of chromosome #12, a CpG site of the sequence from 134597357 to 134602649 on chromosome #10 is located at 134599823 of chromosome #10, a CpG site of the sequence from 144649774 to 144651774 on chromosome #8 is located at 144651002 of chromosome #8, a CpG site of the sequence from 47998899 to 47999517 on chromosome #1 is located at 47999163 of chromosome #1, a CpG site of the sequence from 26394102 to 26396102 on chromosome #2 is located at 26395458 of chromosome #2, a CpG site of the sequence from 104510870 to 104513913 on chromosome #8 is located at 104512877 of chromosome #8, a CpG site of the sequence from 98289604 to 98290404 on chromosome #8 is located at 98290148 of chromosome #8, a CpG site of the sequence from 63281034 to 63281347 on chromosome #2 is located at 63281139 of chromosome #2, a CpG site of the sequence from 67873388 to 67875600 on chromosome #8 is located at 67874178 of chromosome #8, a CpG site of the sequence from 76555366 to 76556079 on chromosome #4 is located at 76555832 of chromosome #4, a CpG site of the sequence from 63782394 to 63790471 on chromosome #1 is located at 63789278 of chromosome #1, a CpG site of the sequence from 7849945 to 7850439 on chromosome #5 is located at 7850070 of chromosome #5, a CpG site of the sequence from 39186777 to 39187968 on chromosome #2 is located at 39187533 of chromosome #2, and a CpG site of the sequence from 74207665 to 74208665 on chromosome #14 is located at 74208165 of chromosome #14.

5. The method of claim 1, wherein the biological sample is one selected from the group consisting of tissue, cells, blood, plasma, stool and urine derived from a patient with suspected liver cancer or a subject diagnosed with liver cancer.

6. The method of claim 1, wherein the step (b) is performed by one method selected from the group consisting of PCR, methylation-specific PCR, real-time methylation-specific PCR, MethyLight PCR, MethyLight digital PCR, EpiTYPER, PCR using methylated DNA-specific binding protein, quantitative PCR, DNA chip assay, pyrosequencing and bisulfite sequencing.

7. The method of claim 1, further comprising:
after step (b), (c) comparing the methylation level with a methylation level in a normal control.

8. A kit for diagnosing a risk of the onset of liver cancer, comprising:
a probe binding to a CpG site at a location selected from the group consisting of the sequence from 25438725 to 25439276 on chromosome #2, the sequence from 95941906 to 95942979 on chromosome #12, the sequence from 134597357 to 134602649 on chromosome #10, the sequence from 144649774 to 144651774 on chromosome #8, the sequence from 47998899 to 47999517 on chromosome #1, the sequence from 26394102 to 26396102 on chromosome #2, the sequence from 104510870 to 104513913 on chromosome #8, the sequence from 98289604 to 98290404 on chromosome #8, the sequence from 63281034 to 63281347 on chromosome #2, the sequence from 67873388 to 67875600 on chromosome #8, the sequence from 76555366 to 76556079 on chromosome #4, the sequence from 63782394 to 63790471 on chromosome #1, the sequence from 7849945 to 7850439 on chromosome #5, the sequence from 39186777 to 39187968 on chromosome #2, and the sequence from 74207665 to 74208665 on chromosome #14.

9. The kit of claim 8, which comprises two or more probes binding to the CpG sites.
